# EUROPEAN PATENT SPECIFICATION

(11) **EP 1 355 588 B1**
(45) Date of publication and mention of the grant of the patent: **15.08.2007**
(21) Application number: 01998066.3
(22) Date of filing: 18.12.2001
(51) Int. Cl.: A61F 2/82

(54) **Device for delivery of therepeutic agents**
Vorrichtung zur Abgabe von therapeutischen Wirkstoffen
Dispositif d'administration des agents therapeutiques

(30) Priority: 22.12.2000 US 258024 P; 13.02.2001 US 782927; 13.02.2001 US 783254; 13.02.2001 US 783253; 13.02.2001 US 782804; 26.07.2001 US 308381 P; 01.11.2001 US 2595
(43) Date of publication of application: 29.10.2003
(73) Proprietor: Avantec Vascular Corporation, Sunnyvale, CA 94086 (US)
(72) Inventor: SIRHAN, Motasim, Sunnyvale, CA 94087 (US); YAN, John, Los Gatos, CA 95032 (US)
(74) Representative: Kazi, Ilya
(86) International application number: PCT/US2001/049366
(87) International publication number: WO 2002/056790

(56) References cited:
- EP-A- 0 923 953
- WO-A-00/10622
- US-A- 5 286 254
- US-A- 5 419 760
- US-A- 5 776 184
- US-A- 6 054 122

## Description

### BACKGROUND OF THE INVENTION

1. Field of the Invention.

The present invention relates generally to medical devices More particularly, the present invention provides luminal prostheses, such as vascular stents and grafts for reducing or inhibiting restenosis.

A number of percutaneous intravascular procedures have been developed for treating stenotic atherosclerotic regions of a patient's vasculature to restore adequate blood flow. The most successful of these treatments is percutaneous transluminal angioplasty (PTA). In PTA, a catheter, having an expandable distal end usually in the form of an inflatable balloon, is positioned in the blood vessel at the stenotic site. The expandable end is expanded to dilate the vessel to restore adequate blood flow beyond the diseased region. Other procedures for opening stenotic regions include directional arthrectomy, rotational arthrectomy, laser angioplasty, stenting, and the like. While these procedures have gained wide acceptance (either alone or in combination, particularly PTA in combination with stenting), they continue to suffer from significant disadvantages. A particularly common disadvantage with PTA and other known procedures for opening stenotic regions is the frequent occurrence of restenosis.

Restenosis refers to the re-narrowing of an artery after an initially successful angioplasty. Restenosis afflicts approximately up to 50% of all angioplasty patients and is the result of injury to the blood vessel wall during the lumen opening angioplasty procedure. In some patients, the injury initiates a repair response that is characterized by smooth muscle cell proliferation referred to as "hyperplasia" in the region traumatized by the angioplasty. This proliferation of smooth muscle cells re-narrows the lumen that was opened by the angioplasty within a few weeks to a few months, thereby necessitating a repeat PTA or other procedure to alleviate the restenosis.

A number of strategies have been proposed to treat hyperplasia and reduce restenosis. Previously proposed strategies include prolonged balloon inflation during angioplasty, treatment of the blood vessel with a heated balloon, treatment of the blood vessel with radiation following angioplasty, stenting of the region, and other procedures. While these proposals have enjoyed varying levels of success, no one of these procedures is proven to be entirely successful in substantially or completely avoiding all occurrences of restenosis and hyperplasia.

As an alternative or adjunctive to the above mentioned therapies, the administration of therapeutic agents following PTA for the inhibition of restenosis has also been proposed. Therapeutic treatments usually entail pushing or releasing a drug through a catheter or from a stent. While holding great promise, the delivery of therapeutic agents for the inhibition of restenosis has not been entirely successful.

Accordingly, it would be a significant advance to provide improved devices
for reducing, inhibiting, or treating restenosis and hyperplasia which may follow angioplasty and other interventional treatments. This invention satisfies at least some of these and other needs.

2. Description of the Background Art

A full description of an exemplary luminal prosthesis for use in the present invention is described in US 6,602,282.
Method and apparatus for releasing active substances from implantable and other devices are described in U.S. Patent Nos. 6,096,070; 5,824,049; 5,624,411; 5,609,629; 5,569,463; 5,447,724; and 5,464,650. The use of stents for drug delivery within the vasculature are described in PCT Publication No. WO 01/01957 and U.S. Patent Nos. 6,099,561; 6,071,305; 6,063,101; 5,997,468; 5,980,551; 5,980,566; 5,972,027; 5,968,092; 5,951,586; 5,893,840; 5,891,108; 5,851,231; 5,843,172; 5,837,008; 5,769,883; 5,735,811; 5,700,286; 5,679,400; 5,649,977; 5,637, 113; 5,591,227; 5,551,954; 5,545,208; 5,500,013; 5,464,450; 5,419,760; 5,411,550; 5,342,348; 5,286,254; and 5,163,952. Biodegradable materials are described in U.S. Patent Nos. 6,051,276; 5,879,808; 5,876,452; 5,656,297; 5,543,158; 5,484,584; 5,176,907; 4,894,231; 4,897,268; 4,883,666; 4,832,686; and 3,976,071. The use of hydrocyclosiloxane as a rate limiting barrier is described in U.S. Patent No. 5,463,010. Methods for coating of stents is described in U.S. Patent No. 5,356,433. Coatings to enhance biocompatibility of implantable devices are described in U.S. Patent Nos. 5,463,010; 5,112,457; and 5,067,491. Energy based devices are described in U.S. Patent Nos. 6,031,375; 5,928,145; 5,735,811; 5,728,062; 5,725,494; 5,409,000, 5,368,557; 5,000,185; and 4,936,281. Magnetic processes, some of which have been used in drug delivery systems, are described in U.S. Patent Nos. 5,427,767; 5,225,282; 5,206,159; 5,069,216; 4,904,479; 4,871,716; 4,501,726; 4,357,259; 4,345,588; and 4,335,094.
WO 04/10622, considered as the closest prior art, shows a coated implantable medical device, adapted for introduction into the vascular system, having a coating layer and a layer of bio-active material positioned on the coating layer, the coating providing for the controlled release of the bio-active material. EP 0 923 953 shows a coating for an implantable device including an undercoat of polymeric material containing a biologically active material and a topcoat which covers less than the entire surface of the undercoat. US 5,286,254 shows a drug delivery apparatus including a device which is positioned in a body passageway. A selected drug is introduced into the device and transported across a drug transport wall of the device.
US-A-577 6 184 discloses a device in which multiple coatings of drug and polymer are applied to a stent to produce a concentration gradient.

### BRIEF SUMMARY OF THE INVENTION

The present invention provides improved devices for inhibiting restenosis and hyperplasia after intravascular intervention. In particular, the present invention provides luminal prostheses which allow for programmed and controlled substance delivery with increased efficiency and/or efficacy to selected locatious within a patient's vasculature to inhibit restenosis. Moreover, the present invention minimizes drug washout and provides minimal to no hindrance to endothelialization of the vessel wall.

The term "intravascular intervention" includes a variety of corrective procedures that may be performed to at least partially resolve a stenotic, restenotic, or thrombotic condition ir a body lumen. Usually, the corrective procedure will comprise balloon angioplasty. The corrective procedure could also comprise directional atherectomy, rotational atherectomy, laser angioplasty, stenting, or the like, where the lumen of the treated blood vessel is enlarged to at least partially alleviate a stenotic condition which existed prior to the treatment.

A device for intracorporeal use according to the present invention is set out in Claim 1. Preferred features of the device are set out in the dependent claims. A luminal delivery prosthesis may comprise a scaffold which is implantable in a body lumen and means on the scaffold for releasing a substance. The substance may be released over a predetermined time pattern comprising an initial phase wherein the substance delivery rate is below a threshold level and a subsequent phase wherein the substance delivery rate is above a threshold level.

The predetermined time pattern of the present invention improves the efficiency of drug delivery by releasing a lower or minimal amount of the substance until a subsequent phase is reached, at which point the release of the substance may be substantially higher. Thus, time delayed substance release can be programmed to impact restenosis substantially at the onset of events leading to .smooth muscle cell proliferation (hyperplasia). The present invention can further minimize substance washout by timing substance release to occur after at least initial cellularization and/or endothelialization which creates a barrier over the stent to reduce loss of the substance directly into the bloodstream. Moreover, the predetermined time pattern may reduce substance loading and/or substance concentration as well as potentially providing minimal to no hindrance to endothelialization of the vessel wall due to the minimization of drug washout and the increased efficiency of substance release.

The scaffold may be in the form of a stent, which additionally maintains luminal patency, or may be in the form of a graft, which additionally protects or enhances the strength of a luminal wall. The scaffold may be radially expansible and/or self expanding and is preferably suitable for luminal placement in a body lumen. The body lumen may be any blood vessel in the patient's vasculature, including veins, arteries, aorta, and particularly including coronary and peripheral arteries, as well as previously implanted grafts, shunts, fistulas, and the like. It will be appreciated that the present invention may also be applied to other body lumens as well as to many internal corporeal tissue organs, such as organs, nerves, glands, ducts, and the like. An exemplary stent for use in the present invention is described in US 6,602,282.

It will be appreciated that the above-described benefits of time delayed release allow for a wide array of substances to be effectively delivered. The substance may comprise at least one agent selected from the group consisting of immunosuppressant agent, anti-inflammatory agent, anti-proliferative agent, anti-migratory agent, anti-fibrotic agent, antithrombotic agent, anti-platelet agent, and IIb/IIIa agent. Preferably, the agent is an immunosuppressant agent selected from the group consisting of mycophenolic acid, rapamyacin, cyclosporine A, cycloheximide, cyclophosphamide, mizoribine, methylprednisolone, azathioprine, ribovirin, FK506, tiazofurin, methotrexate, zafurin, and mycophenolate mofetil. The total amount of substance released will typically be in a range from 1 g. to 2000 g., preferably in a range from 10 g. to 1000 g., most preferably in a range from 50 g. to 500 g. The release rate during the initial phase will typically be from 0 g/day to 50 g/day, usually from 5 g/day to 30 g/day. The substance release rate during the subsequent phase will be much higher, typically being in the range from 5 g/day to 200 g/day, usually from 10 g/day to 100 g/day. Thus, the initial release rate will typically be from 0 % to 99 % of the subsequent release rates, usually from 0 % to 90 %, preferably from 0 % to 75 %. Of course, the release rates may vary during either or both of the initial and subsequent release phases. There may also be additional phase(s) for release of the same substance(s) and/or different substance(s).

The duration of the initial, subsequent, and any other additional phases may vary. Typically, the initial phase will be sufficiently long to allow initial cellularization or endothelialization of at least part of the stent, usually being less than 12 weeks, more usually from 1 hour to 8 weeks, more preferably from 12 hours to 2 weeks, most preferably from 1 day to 1 week. The durations of the subsequent phases may also vary, typically being from 4 hours to 24 weeks, more usually from 1 day to 12 weeks, more preferably in a time period of 2 days to 8 weeks in a vascular environment, most preferably in a time period of 3 days to 50 days in a vascular environment.

The present invention is directed to improved devices for preparation or treatment of susceptible tissue sites. As used herein, susceptible tissue site refers to a tissue site that is injured, or may become injured as a result of an impairment (e.g., disease, medical condition), or may become injured during or following an interventional procedure such as an intravascular intervention. The term "intravascular intervention" includes a variety of corrective procedures that may be performed to at least partially resolve a stenotic, restenotic, or thrombotic condition in a blood vessel, usually an artery, such as a coronary artery. Usually, the corrective procedure will comprise balloon angioplasty. The corrective procedure may also comprise directional atherectomy, rotational atherectomy, laser angioplasty, stenting, or the like, where the lumen of the treated blood vessel is enlarged to at least partially alleviate a stenotic condition which existed prior to the treatment. The susceptible tissue site may include tissues associated with intracorporeal lumens, organs, or localized tumors. In one embodiment, the present devices and methods reduce the formation or progression of restenosis and/or hyperplasia which may follow an intravascular intervention. In particular, the present invention is directed to corporeal, in particular intracorporeal devices and methods using the same.

As used herein, the term "intracorporeal body" refers to a body lumens or internal corporeal tissues and organs, within a corporeal body. The body lumen may be any blood vessel in the patient's vasculature, including veins, arteries, aorta, and particularly including coronary and peripheral arteries, as well as previously implanted grafts, shunts, fistulas, and the like. It will be appreciated that the present invention may also be applied to other body lumens, such as the biliary duct, which are subject to excessive neoplastic cell growth. Examples of internal corporeal tissues and organs, include various organs, nerves, glands, ducts, and the like. In an embodiment, the device includes luminal prostheses such as vascular stents or grafts. In another embodiment, the device may include, cardiac pacemaker leads or lead tips, cardiac defibrillator leads or lead tips, heart valves, sutures, or needles, pacemakers, orthopedic devices, appliances, implants or replacements, or portions of any of the above.

In an embodiment, the devices of the present invention, reduce and/or inhibit the occurrence of restenosis (defined as when the artery narrows greater than about 40 to about 80% of the acute vessel diameter achieved by the vascular intervention, such as stenting, usually from about 50 to about 70%) while allowing for the generation of small amount of cellularization, endothelialization, or neointima, preferably, in a controlled manner.

In an embodiment, the device includes a structure and at least one source of at least one therapeutic capable agent associated with the structure. In an embodiment, the structure, may be an expandable structure. In another embodiment, the structure may have a substantially constant size or diameter, or alternatively depending on the application and use, may be a contractable structure. In an embodiment, the structure includes at least one surface, usually, a tissue facing surface. In another embodiment, the structure includes a tissue facing surface and another surface, usually a lumen facing surface. In an embodiment, the structure may have an interior disposed between two surfaces, usually, the tissue facing and the lumen facing surfaces.

The device may include an expandable structure implantable within a corporeal body which includes the susceptible tissue site. The device, alternatively or additionally, may be an implantable device configured for implanting with or without expansion at a targeted corporeal site. The targeted corporeal site may include the susceptible tissue site or may be a site (e.g., other body organs or lumens), for example a targeted intracorporeal site such as an artery, which supplies blood to the susceptible tissue site. In an embodiment, the expandable structure may be in the form of a stent, which additionally maintains luminal patency, or in the form of a graft, which additionally protects or enhances the strength of a luminal wall. The device, may comprise at least in part, a scaffold formed from an open lattice or an at least substantially closed surface. In an embodiment, the stent comprises a scaffold formed at least in part from an open lattice. The expandable structure may be radially expandable and/or self-expanding and is preferably suitable for luminal placement in a body lumen.

The expandable structure may be formed of any suitable material such as metals, polymers, or a combination thereof In one embodiment, the expandable structure may be formed of an at least partially biodegradable material, selected from the group consisting of polymeric material, metallic materials, or combinations thereof. The at least partially biodegradable material, preferably degrades over time. Examples of polymeric material include Poly-L-lactic acid, having a delayed degradation to allow for the recovery of the vessel before the structure is degraded. Example of metallic material include metals or alloys degradable in the corporeal body, such as stainless steel. An exemplary stent for use in the present invention is described in US 6,602,282.

The therapeutic capable agent is associated at least in part with the structure in a manner as to become available, immediately or after a delay period, to the susceptible tissue site upon introduction of the device within or on the corporeal body. As used herein the term "associated with" refers to any form of association such as directly or indirectly being coupled to, connected to, disposed on, disposed within, attached to, adhered to, bonded to, adjacent to, entrapped in, absorbed in, absorbed on, and like configurations.

The source may be disposed or formed adjacent at least a portion of the structure. In an embodiment, the source may be disposed or formed adjacent at least a portion of the structure. In one embodiment, the source may be disposed or formed adjacent at least a portion of either or both surfaces of the expandable structure, within the interior of the structure disposed between the two surfaces, or any combination thereof. The association of the therapeutic capable agent with the structure may be continuous or in discrete segments.

In one embodiment, a luminal prosthesis makes available one or more therapeutic capable agents to one or more selected locations within a patient's vasculature, including the susceptible tissue site, to reduce the formation or progression of restenosis and/or hyperplasia. As used herein, therapeutic capable agent includes compounds that are either therapeutic as they are introduced to the subject under treatment, become therapeutic after entering the corporeal body of the subject upon reaction with a native substance or condition, or another introduced substance or condition. As used herein, the term "made available" means to have provided the substance (e.g., therapeutic capable agent) at the time of release or administration, including having made the substance available at a corporeal location such as an intracorporeal location or target site, regardless of whether the substance is in fact delivered, used by, or incorporated into the intended site, such as the susceptible tissue site.

The delivery of the therapeutic capable agent to the susceptible tissue site, or making the therapeutic capable agent available to the susceptible tissue site, may be direct, or indirect through another corporeal site. In an embodiment the another corporeal site is a targeted intracorporeal site, for example an intracorporeal lumen, such as an artery, supplying blood to the susceptible tissue site.

As used herein the therapeutic capable agent includes at least one compound molecular species, and/or biologic agent which is either therapeutic as it is introduced to the corporeal body (e.g., human subject) under treatment, or becomes therapeutic after entering the corporeal body of the subject (or exposed to the surface of the corporeal body as the case may be), by for example, reaction with a native or non-native substance or condition. Examples of native conditions include pH (e.g. acidity), chemicals, temperature, salinity osmolality, and conductivity; with non-native conditions including those such as magnetic fields, electromagnetic fields (such as radiofrequency and microwave) and ultrasound. In the present application, the chemical name of any of the therapeutic capable agents or other compounds is used to refer to the compound itself and to pro-drugs (precursor substances that are converted into an active form of the compound in the body), and/or pharmaceutical derivatives, analogues, or metabolites thereof (bioactive compound to which the compound converts within the body directly or upon introduction of other agents or conditions (e.g., enzymatic, chemical, energy), or environment (e.g., pH)).

The therapeutic capable agent may be selected from a group consisting of immunosuppressants, anti-inflammatories, anti-proliferatives, anti-migratory agents, anti-fibrotic agents, proapoptotics, calcium channel blockers, anti-neoplastics, antibodies, antithrombotic agents, anti-platelet agents, IIb/IIIa agents, antiviral agents, and a combination thereof.

Specific examples of therapeutic capable agent include: mycophenolic acid, mycophenolate mofetil, mizoribine, methylprednisolone, dexamethasone, rapamycin LY294002, (FK 506), cyclophosphamide, cyclosporine, daclizumab, azathioprine, prednisone, derivatives and combinations thereof.

In an embodiment, the source of the therapeutic capable agent is a polymeric material including therapeutic capable agent moieties as a structural subunit of the polymer. The therapeutic capable agent moieties are polymerized and associated to one another through suitable linkages (e.g. ethylenic) forming polymeric therapeutic capable agent. Once the polymeric therapeutic capable agent is brought into contact with tissue or fluid such as blood, the polymeric therapeutic capable agent subunits disassociate. Alternatively, the therapeutic capable agent may be released as the polymeric therapeutic capable agent degrades or hydrolyzes, preferably, through surface degradation or hydrolysis, making the therapeutic capable agent available to the susceptible tissue site, preferably over a period of time. Examples of methods and compounds for polymerizing therapeutic capable agents are described in WO 99/12990 Patent Application by Kathryn Uhrich, entitled "Polyanhydrides With Therapeutically Useful Degradation Products," and assigned to Rutgers University, An example of a therapeutic capable agents and a suitable reaction ingredient unit includes, mycophenolic acid with adipic acid and/or salicylic acid in acid catalyzed esterification reaction; mycophenolic acid with aspirin and/or adipic acid in acid catalyzed esterification reaction, mycophenolic acid with other NSAIDS, and/or adipic acid in acid catalyzed esterification reaction. In an embodiment, the polymeric therapeutic capable agent may be associated with a polymeric and/or metallic backbone.

The devices of the present invention may be configured to release or make available the therapeutic capable agent at one or more phases, the one or more phases having similar or different performance (e.g., release) profiles. The therapeutic capable agent may be made available to the tissue at amounts which may be sustainable, intermittent, or continuous; in one or more phases and/or rates of delivery; effective to reduce any one or more of smooth muscle cell proliferation, inflammation, immune response, hypertension, or those complementing the activation of the same. Any one of the at least one therapeutic capable agents may perform one or more functions, including preventing or reducing proliferative/restenotic activity, reducing or inhibiting thrombus formation, reducing or inhibiting platelet activation, reducing or preventing vasospasm, or the like.

The total amount of therapeutic capable agent made available to the tissue depends in part on the level and amount of desired therapeutic result. The therapeutic capable agent may be made available at one or more phases, each phase having similar or different release rate and duration as the other phases. The release rate may be pre-defined. In an embodiment, the rate of release may provide a sustainable level of therapeutic capable agent to the susceptible tissue site. In another embodiment, the rate of release is substantially constant. The rate may decrease and/or increase over time, and it may optionally include a substantially non-release period. The release rate may comprise a plurality of rates. In an embodiment the plurality of release rates include at least two rates selected from the group consisting of substantially constant, decreasing, increasing, substantially non-releasing.

The total amount of therapeutic capable agent made available or released will typically be in an amount ranging from about 0.1 ug to about 10 g, generally from about 0.1 ug to about 10 mg, preferably from about 1 ug to about 10 mg, more preferably from about 1 ug to about 2 mg, from 10 ug to about 2 mg, or from about 50 ug to about 1 mg.

In an embodiment, the therapeutic capable agent may be released in a time period, as measured from the time of implanting of the device, ranging from about 1 day to about 200 days; from about 1 day to about 45 days; or from about 7 days to about 21 days.

In an embodiment the release rate of the therapeutic capable agent per day may range from about 0.001 micrograms (ug) to about 200 ug, preferably, from about 0.5 ug to about 200 ug, and most preferably, from about 1 ug to about 60 ug.

The therapeutic capable agent may be made available at an initial phase and one or more subsequent phases. When the therapeutic capable agent is delivered at different phases, the initial delivery rate will typically be from about 0 to about 99 % of the subsequent release rates, usually from about 0 % to about 90 %, preferably from about 0 % to 75 %. In an embodiment a mammalian tissue concentration of the substance at an initial phase will typically be within a range from about 0.001 nanogram (ng)/mg of tissue to about 100 ug/mg of tissue; from about 1 ng/mg of tissue to about 100 ug/mg of tissue; from about 1 ng/mg of tissue to about 10 ug/mg of tissue. A mammalian tissue concentration of the substance at a subsequent phase will typically be within a range from about 0.001 ng/mg of tissue to about 600 ug/mg of tissue, preferably from about 1 ng/mg of tissue to about 10 ug/mg of tissue.

The rate of delivery during the initial phase will typically range from about 0.001 ng to about 50 ug per day, usually from about 0.1 ug to about 30 ug per day, more preferably, from about 1 ug per day to about 20 ug per day. The rate of delivery at the subsequent phase may range from about 0.01 ug per day to about 200 ug per day, usually from about lug per day to about 100 ug per day. In one embodiment, the therapeutic capable agent is made available to the susceptible tissue site in a programmed and/or controlled manner with increased efficiency and/or efficacy. Moreover, the present invention provides limited or reduced hindrance to endothelialization of the vessel wall.

The duration of the initial, subsequent, and any other additional phases may vary. For example, the release of the therapeutic capable agent may be delayed from the initial implantation of the device. Typically the delay is sufficiently long to allow the generation of sufficient cellularization or endothelialization at the treated site to inhibit loss of the therapeutic capable agent into the vascular lumen.. Typically, the duration of the initial phase will be sufficiently long to allow initial cellularization or endothelialization at, at least part of the device. Typically, the duration of the initial phase whether being a delayed phase or a release phase, is usually less than about 12 weeks, more usually from about 1 hour to about 8 weeks, more preferably from about 12 hours to about 4 weeks, from about 12 hours to about 2 weeks, from about 1 day to about 2 weeks, or from about 1 day to about 1 week.

The durations of the one or more subsequent phases may also vary, typically being from about 4 hours to about 24 weeks, from about 1 day to about 12 weeks, from about 2 days to about 8 weeks, more preferably in from about of 3 days to about 50 days. In an embodiment, the duration specified relates to a vascular environment. The more than one phase may include similar or different durations, amounts, and/or rates of release. For example, in one scenario, there may be an initial phase of delay, followed by a subsequent phase of release a first subsequent rate, and second subsequent phase at a second subsequent rate of release, and the like.

In an embodiment, the device further includes another compound, such as another therapeutic capable agent, or another compound enabling and/or enhancing either or both the release and efficacy of the therapeutic capable agent. The another therapeutic capable agent may be associated with expandable structure in the same or different manner as the first therapeutic capable agent.

The another therapeutic capable agent may act in synergy with the therapeutic capable agent, in ways such as compensating for the possible reactions and by-products that can be generated by the therapeutic capable agent. By way of example, the therapeutic capable agent may reduce generation of desired endothelial cells, thus by including a suitable another therapeutic capable agent, more endothelialization may be achieved.

The another therapeutic capable agent may comprise at least one compound selected from the group consisting of anti-cancer agents; chemotherapeutic agents; thrombolytics; vasodilators; antimicrobials or antibiotics antimitotics; growth factor antagonists; free radical scavengers; biologic agents; radiotherapeutic agents; radiopaque agents; radiolabelled agents; anti-coagulants such as heparin and its derivatives; anti-angiogenesis drugs such as Thalidomide^{™}; angiogenesis drugs; PDGF-B and/or EGF inhibitors; anti-inflamatories including psoriasis drugs; riboflavin; tiazofurin; zafurin; anti-platelet agents including cyclooxygenase inhibitors such as acetylsalicylic acid, ADP inhibitors such as clopidogrel (e.g., Plavix^{™}) and ticlopdipine (e.g., ticlid™), phosphodiesterase III inhibitors such as cilostazol (e.g., Pletal™), glycoprotein IIb/IIIa agents such as abciximab (e.g., Rheopro™); eptifibatide (e.g., Integrilin™), and adenosine reuptake inhibitors such as dipyridmoles; healing and/or promoting agents including anti-oxidants, nitrogen oxide donors; antiemetics; antinauseants; derivatives and combinations thereof.

The another therapeutic agent may be released prior to, concurrent with, or subsequent to, the therapeutic capable agent, at similar or different rates and phases.

In an embodiment, the another compound comprises, an enabling compound responsive to an external form of energy, or native condition, to effect or modify the release of the therapeutic capable agent. The respondable compound may be associated with the therapeutic capable agent, the rate-controlling element, the expandable structure, or a combination thereof. The second enabling compound may be formed from magnetic particles coupled to the therapeutic capable agent. The energy source may be a magnetic source for directing a magnetic field at the prosthesis after implantation to effect release of the therapeutic capable agent.

In an embodiment, the source includes a rate-controlling element for affecting the rate of release of the therapeutic capable agent and/or the another compound.

In an embodiment, the rate-controlling element may be disposed or formed adjacent the structure. In one embodiment, the rate-controlling element may be disposed or formed adjacent at least a portion of the optional one or more surfaces of the structure (e.g., luminal or tissue facing surfaces), or within the optional interior of the structure, or any combination thereof. The therapeutic capable agent or the optional another compound may be disposed adjacent the rate-controlling element. Additionally and/or alternatively, in one embodiment, the therapeutic capable agent or the optional another compound may be disposed within the rate-controlling element forming a matrix therewith. In an embodiment, the therapeutic capable agent or the optional another compound itself is a rate-controlling element, as for example, when the therapeutic capable agent or the optional another compound is a polymeric material.

The term matrix as used herein refers to an association between the rate-controlling element and the therapeutic capable agent (or the optional another compound) and/or the therapeutic capable agent (or the optional another compound) and any other compounds or structures affecting the release of the therapeutic capable agent. In an embodiment, the matrix is formed as a matrix interface between the rate-controlling element and the therapeutic capable agent and/or the optional another compound. In an embodiment, the rate-controlling element may comprise multiple adjacent layers formed from the same or different material. The therapeutic capable agent or the optional another compound may be present adjacent one or more of the rate-controlling element layers. Additionally and/or alternatively, the therapeutic capable agent or the optional another compound may form a matrix and/or matrix interface with one or more of the rate-controlling element layers.

In another embodiment, when the rate-controlling element is present as multiple layers, the any one of the more than one layers may include independently none, one, or more of the plurality of compounds (e.g., the at least one therapeutic capable agent, another compound. Each of the plurality of compounds such as the another compound and/or more than one therapeutic capable agent, may form a different matrix with the rate-controlling element. In an embodiment, as further, described below, the therapeutic capable agent may form the matrix, as when the therapeutic capable agent is a polymeric therapeutic capable agent, thus controlling the release of the active component to the susceptible tissue site. Alternatively, or additionally, the rate-controlling element may be another compound, such as another therapeutic capable agent which can have an impact on the release rate of the first therapeutic capable agent.

The therapeutic capable agent may be associated with either or both the structure (e.g., expandable structure) and the rate-controlling element in one or more ways as described above. The therapeutic capable agent may be disposed adjacent (e.g., on or within) the expandable structure. Alternatively or additionally, the therapeutic capable agent may be disposed adjacent (e.g., on or within) the rate-controlling element, or in an interface between structure and the rate-controlling element, in a pattern that provides the desired performance (e.g., release rate). In an embodiment, the device includes an outer layer including the therapeutic capable agent. In an embodiment, the therapeutic capable agent outer layer provides for a bullous release of the therapeutic capable agent upon introduction of the device to the corporeal body.

The rate-controlling element may be formed of a non-degradable, partially degradable, substantially degradable material, or a combination thereof. The material may be synthetic or natural; non-polymeric, polymeric or metallic; or a combination thereof. By way of examples, a metallic material that at least partially degrades with time may be used as the rate-controlling element; as well as non-polymers having large molecular weight, polar or non-polar functional groups, electrical charge, steric hindrance groups, hydrophobic, hydrophilic, or amphiphilic moieties.

Suitable biodegradable rate-controlling element materials include, but are not limited to, poly(lactic acid), poly(glycolic acid) and copolymers, poly dioxanone, poly (ethyl glutamate), poly (hydroxybutyrate), polyhydroxyvalerate and copolymers, polycaprolactone, polyanhydride, poly(ortho esters); poly (iminocarbonates), polycyanoacrylates, polyphosphazenes, copolymers and other aliphatic polyesters, or suitable copolymers thereof including copolymers of poly-L-lactic acid and poly-e-caprolactone; mixtures, copolymers, and combinations thereof.

Suitable nondegradable or slow degrading rate-controlling element materials include, but are not limited to, polyurethane, polyethylenes imine, cellulose acetate butyrate, ethylene vinyl alcohol copolymer, silicone, polytetrafluorethylene (PTFE), parylene, parylast, poly (methyl methacrylate butyrate), poly-N-butyl methacrylate, poly (methyl methacrylate), poly 2-hydroxy ethyl methacrylate, poly ethylene glycol methacrylates, poly vinyl chloride, poly(dimethyl siloxane), poly(tetrafluoroethylene), poly (ethylene oxide), poly ethylene vinyl acetate, poly carbonate, poly acrylamide gels, N-vinyl-2-pyrrolidone, maleic anhydride, Nylon, cellulose acetate butyrate (CAB) and the like, including other synthetic or natural polymeric substances; mixtures, copolymers, and combinations thereof. In an embodiment the rate-controlling element is formed from a material selected from the group consisting of silicone, polytetrafluoroethylene, parylast, polyurethane, parylene, cellulose acetate butyrate; mixtures, copolymers and combinations thereof.

Suitable natural material include: fibrin, albumin, collagen, gelatin, glycosoamimoglycans, oligosaccharides & poly saccharides, chondroitin, phosholipids, phosphorylcholine, glycolipids, proteins, amino acids, cellulose, and mixtures, copolymers, or combinations thereof. Other suitable material include, titanium, chromium, Nitinol, gold, stainless steel, metal alloys, or a combination thereof; and other compounds that may release the therapeutic capable agent as a result of interaction (e.g., chemical reaction, high molecular weight, steric hindrance, hyrophobicity, hydrophilicity, amphilicity, heat) of the therapeutic capable agent with the rate-controlling element material (e.g, a non-polymer compound). By way of example, a combination of two or more metals or metal alloys with different galvanic potentials to accelerate corrosion by galvanic corrosion pathways may also be used.

In another embodiment, the surface of the structure may be pre-processed using any of a variety of procedures, including, cleaning; physical modifications such as etching or abrasion; and chemical modifications such as solvent treatment, the application of primer coatings, the application of surfactants, plasma treatment, ion bombardment, and covalent bonding. In an embodiment, a metal film or alloy with a small pits or pin holes to accelerate corrosion by pitting corrosion, allowing the pin hole formed by the corrosion to act as an orifice for drug release. In an embodiment, the therapeutic capable agent may be attached to the metal or metal alloy.

The degradable material may degrade by bulk degradation or hydrolysis. In an embodiment, the rate-controlling element degrades or hydrolyzes throughout, or preferably, by surface degradation or hydrolysis, in which a surface of the rate-controlling element degrades or hydrolyzes over time while maintaining bulk integrity. In another embodiment, hydrophobic rate-controlling elements are preferred as they tend to release therapeutic capable agent at desired release rate. A non-degradable rate-controlling element may release therapeutic capable agent by diffusion. By way of example, if the rate-controlling element is formed of non-polymeric material, the therapeutic capable agent may be released as a result of the interaction (e.g., chemical reaction, steric hinderence, hyrophobicity, hydrophilicity, amphilicity) of the therapeutic capable agent with the rate-controlling element material (e.g, a non-polymer compound). In an embodiment, when the rate-controlling element does not form, at least a sufficient matrix with the therapeutic capable agent, the therapeutic capable agent may be released by diffusion through the rate-controlling element.

In yet another embodiment the therapeutic capable agent is made available to the susceptible tissue site as the native environment of the area where the device is implanted changes. For example, a change in the pH of the area where the device is implanted may change over time so as to bring about the release of the therapeutic capable agent directly (as for example when a polymeric drug acts as the matrix including both the therapeutic capable agent and the rate-controlling element), or indirectly by affecting the erosion or diffusion characteristic of the rate-controlling element as either or both the matrix or non-matrix. For example, as the pH increases or decreases, the erosion of the rate-controlling element changes allowing for initial and subsequent phase releases.

The rate-controlling element may have a sufficient thickness so as to provide the desired release rate of the therapeutic capable agent. The rate-controlling element will typically have a total thickness in a range from about 10 nm to about 100 um. The thickness may also range from about 50 nm to about 100 um, from about 100 nm to about 50 um, or from about 100 nm to 10 um.

Furthermore, a biocompatible (e.g., blood compatible) layer may be formed over the source and/or the most outer layer of the device, to make or enhance the biocompatibility of the device. Suitable biocompatible material for use as the biocompatible layer include, but are not limited to, polyethylene glycol (PEG), polyethylene oxide (PEO), hydrogels, silicone, polyurethanes, heparin coatings.

The source may be associated with at least a portion of the structure (e.g., prosthesis) using coating methods such as spraying, dipping, deposition, painting, chemical bonding. Such coatings may be uniformly or intermittently applied to structure or may be applied in a random or pre-determined pattern. In an embodiment, when the structure includes one or more surfaces and optional interior between the surfaces, the coating may be applied to only one of the surfaces of the prosthesis or the coating may be thicker on one side.

When the device includes the source including a plurality of compounds (e.g., first therapeutic capable agent and an another compound such as another therapeutic capable agent or enabling compound), the plurality of compounds may be released at different times and/or rates, from the same or different layers when present. Each of the plurality of compounds may be made available independently of another, simultaneous with, or subsequent to the interventional procedure, and may be simultaneous or sequential with one another. For example, a first therapeutic capable agent (e.g., Triptolide^{™} may be released within a time period of 1 day to 45 days with the second therapeutic capable agent (e.g, mycophenolic acid) released within a time period of 2 days to 3 months, from the time of interventional procedure.

The devices of the present invention may be provided together with instructions for use (IFU), separately or as part of a kit. The kit may include a pouch or any other suitable package, such as a tray, box, tube, or the like, may be used to contain the device and the IFU, where the IFU may be printed on a separate sheet or other media of communication and/or on the packaging itself. In an embodiment of a kit, the kit may also include a mounting hook such as a crimping device and/or an expansible inflation member which may be permanently or releaseably coupled to the device of the present invention. In an embodiment, the kit may comprise the device and an IFU regarding the use of a second compound prior to, concurrent with, or subsequent to, the interventional procedure, and optionally the second compound. In an embodiment, the kit comprises the device and the second compound with or without the IFU for the second compound and/or the device.

In one embodiment, the second compound, may be a therapeutic capable agent, an another compound (e.g., the another therapeutic capable agent and/or the another enabling and/or enhancing compound), or a bio-active compound such as an anti-nausea drug; and being similar or different than that made available to the susceptible tissue site by the device; may be administered prior to, concurrent with, or subsequent to the implanting of the device (e.g., prosthesis) of the present invention.

The second compound may be administered from a pathway similar to or different than that used for the delivery of the therapeutic capable agent as part of the device. By way of example, the second compound may be in the form of a tablet to be taken orally, a transdermal patch to be placed on the patient's skin, subcutaneously, systemically by direct introduction to the blood stream, by way of inhalation, or through any other pathways and bodily orifices. Alternatively, the second compound may be made available to the intracorporeal body by a catheter. In an embodiment, the balloon of a balloon catheter (e.g., perfusion), may be used to perfuse the second compound (e.g., perfusion catheter) into the corporeal body or may be coated with the second compound. The second compound may be made available to the patient continuously or in discrete intervals, prior to, concurrent with, or subsequent to the interventional procedure.

The duration of the availability of the second compound usually may be shorter as compared to that of the therapeutic capable agent. In an embodiment, the another compound may be administered to the patient in a time period ranging from about 200 days prior to about 200 days after the interventional procedure, from about 30 days prior to about 30 days after the interventional procedure, from about 1 day prior to about 30 days after the interventional procedure, from about 200 days prior to about up to the interventional procedure, from about 3 months prior to about up to the interventional procedure, or from about 7 days to about 24 hours prior to the interventional procedure. The duration of the availability of the second compound as measured in the patient's blood may range from about 1 hour to about 120 days, from about 12 hours to about 60 days, or from about 24 hours to about 30 days. Examples of bioactive compounds include: antiemetics such as ondansetron (e.g., Zofran™), antinauseant such as dronabinol (e.g., Marinol^{™}) and ganisetron.Hcl (Kytril™).

In one embodiment, the second compound may be the same as the therapeutic capable agent of the device to provide a desired bullous level (e.g., an initial level) of the therapeutic capable agent in the corporeal body. The total amount made available to the susceptible tissue site from the device and the second compound will typically be in a range from about 0.1 ug to about 10 milligrams (mg), preferably in a range from about 10 ug to about 2 mg, more preferably in a range from about 50 ug to about 1.0 mg. In an embodiment the amount of the second compound administered to the patient on a single dose or daily basis, ranges from about 0.5 mg to about 5 g, from about 1 mg to about 3 g, from about 1 g to about 1.5 g, from about 2 g to about 3 g. Examples second compounds being provided at the latter series of doses include, mycophenolic acid, rapamycin; and their respective pro-drugs, metabolites, derivatives, and combinations thereof. In an example mycophenolic acid or rapamycin may be provided as a second compound at individual doses ranging from about 1 g to about 1.5 g, and from about 1 mg to about 3 mg, respectively; and at a daily dose ranging from about 2 g to about 3 g, and from about 2 mg to about 6 mg, respectively.

In operation, methods of delivering the therapeutic capable agents to the susceptible tissue site, comprise positioning the source of the therapeutic capable agent within the intracorporeal site such as the vascular lumen. The therapeutic capable agent is released and/or made available to the susceptible tissue site. In an embodiment, the releasing of the therapeutic capable agent occurs at a pre-determined time period following the positioning of the source. The delay in the release of the therapeutic capable agent may be for a sufficiently long period of time to allow sufficient generation of intimal tissue to reduce occurrence of thrombotic event. The device may comprise a rate-controlling element. In an embodiment the source includes the rate-controlling element. In one embodiment, the releasing of the therapeutic capable agent may occur by surface degradation or hydrolysis of the source. In yet another embodiment, the release of the therapeutic capable agent may occur by bulk degradation of the source. In another embodiment, the releasing the therapeutic capable agent may occur by diffusion through the source. In an embodiment a device including a source of therapeutic capable agent and incorporating any one or more features of the present invention is delivered to a corporeal site such as an intracorporeal body (e.g., body lumen). The corporeal site may be a targeted corporeal site (such as a targeted intracorporeal site), which includes the susceptible tissue site, or a targeted site directly or indirectly providing the therapeutic capable agent to the susceptible tissue site. The therapeutic capable agent is made available to the susceptible tissue site, preferably, in a controlled manner over a period of time.

Methods of treatment, generally, include positioning the source including the at least one therapeutic capable agent and/or optional another compound within the intracorporeal body, concurrently with, or subsequent to, an interventional treatment. More specifically, the therapeutic capable agent may be delivered to a targeted corporeal site (e.g., targeted intracorporeal site) which includes the susceptible tissue site or a targeted site providing the therapeutic capable agent to the susceptible tissue site, concurrently with or subsequent to the interventional treatment. By way of example, following the dilation of the stenotic region with a dilatation balloon, a device (such as a stent) according to the present invention, is delivered and implanted in the vessel. The therapeutic capable agent may be made available to the susceptible tissue site at amounts which may be sustainable, intermittent, or continuous; at one or more phases and/or rates of delivery.

In an embodiment, the release of the therapeutic capable agent to the susceptible tissue site may be delayed. During the delay period none to small amounts of therapeutic capable agent may be released before the release of substantial amount of therapeutic capable agent. Typically the delay is sufficiently long to allow the sufficient generation of intimal tissue or cellularization, at the treated site to reduce occurrence of thrombotic event.

In one embodiment, delay is sufficiently long to allow the generated neointima to cover at least partially the implanted expandable structure. In an embodiment, the therapeutic capable agent may be released in a time period, as measured from the time of implanting of the device, ranging from about 1 day to about 200 days; from about 1 day to about 45 days; or from about 7 days to about 21 days. In an embodiment, the method further includes directing energy at the device to effect release of the therapeutic capable agent from the device. The energy may include one or more of ultrasound, magnetic resonance imaging, magnetic field, radio frequency, temperature change, electromagnetic, x-ray, heat, vibration, gamma radiation, or microwave. In an embodiment, the therapeutic capable agent may be released at a total amount ranging from about 0.1 ug to about 10 g, from about 0.1 ug to about 10 mg, from about 1 ug to about 10 mg, from about 1 ug to about 2 mg, from about 10 ug to about 2 mg, or from about 50 ug to about 1 mg.

In another embodiment of a method of treatment, the releasing includes release of at least one another compound, as described. The anther compound may be another therapeutic capable agent or an enabling compound, as described. The another compound may be released prior to, concurrent with, subsequent to the therapeutic capable agent, or sequentially with the therapeutic capable agent.

In an embodiment, a second compound, as described, may be administered to the patient, prior to, concurrent with, or subsequent to the interventional procedure. The second compound may be administered from pathways, at time periods, and at levels, as described.

### BRIEF DESCRIPTION OF THE DRAWINGS

FIGS. 1A through 1C are cross-sectional views of a device embodying features of the present invention and implanted in a body lumen.

FIGS. 2A through 2N are cross-sectional views of various embodiments of the delivery prosthesis of FIGS. 1A-1C taken along line 2-2.

FIG. 3 is a schematic representation of an exemplary stent for use as the device of the present invention.

FIG. 4 is a graphical representation of the release of a therapeutic capable agent over a predetermined time period.

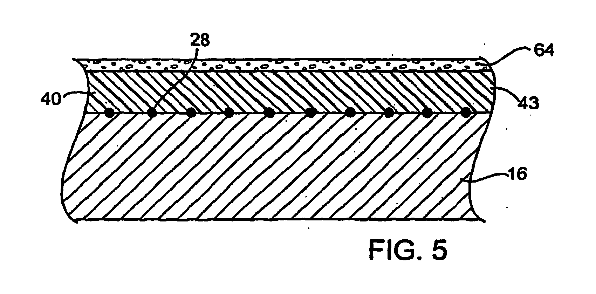

FIG. 5 is a partial cross-sectional view of an embodiment of the prosthesis of FIGS. 1A-1C having a cellular growth thereon after being implanted.

FIGS. 6A through 6I illustrate features of an exemplary method for positioning the prosthesis of FIGS. 1A-1C in a blood vessel.

FIGS. 7A, 7B, 8A, 8B, 9A through 9E, 10A, 10B, 11A, and 11B are graphical representations of the performance of various therapeutic capable agents.

### DETAILED DESCRIPTION OF THE INVENTION

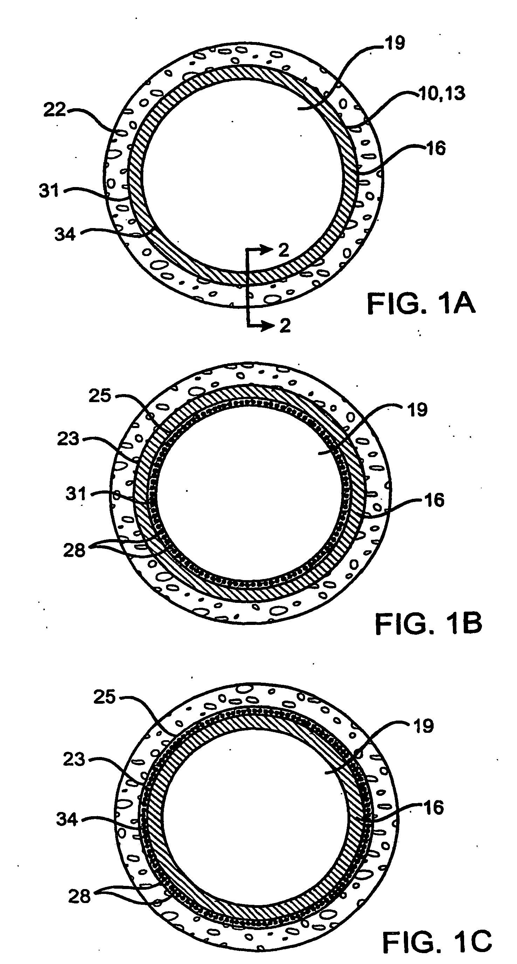

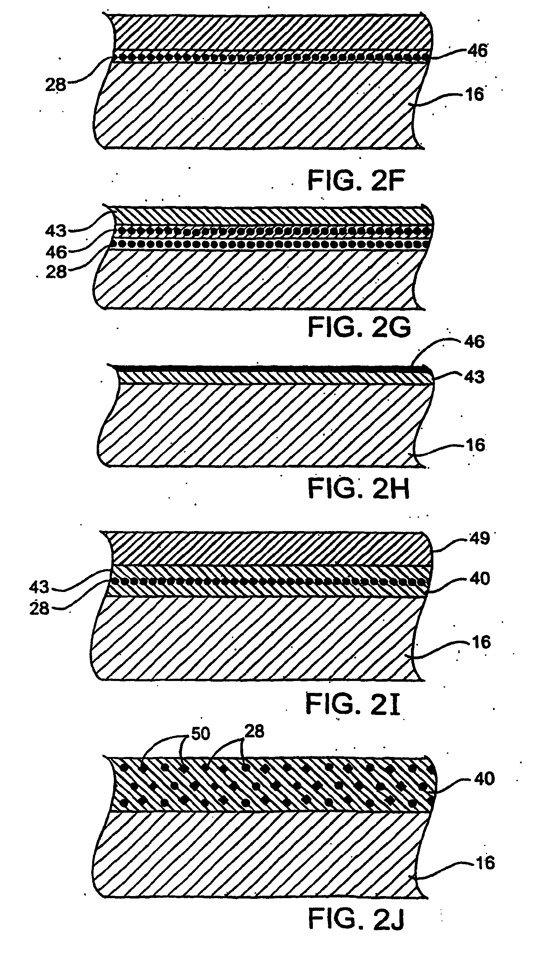

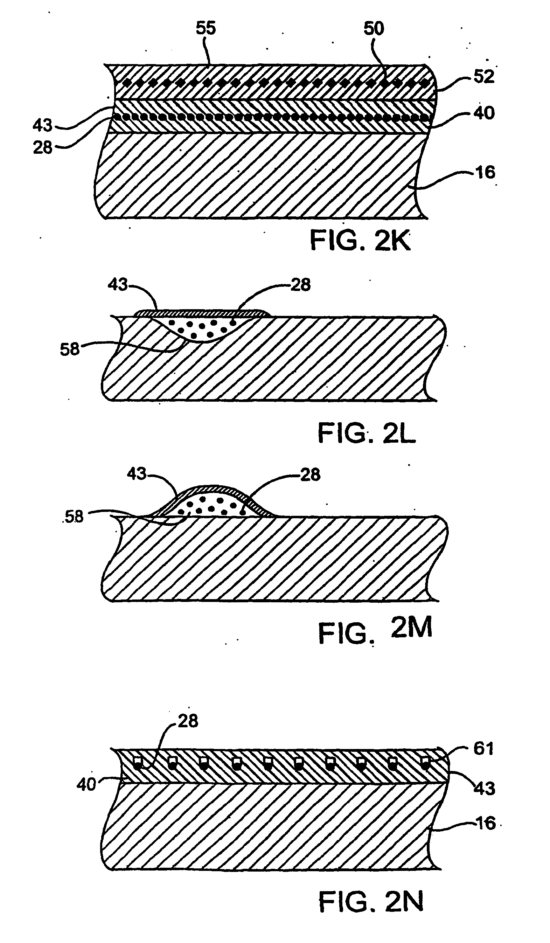

FIGS. 1A-1C, and cross-sectional drawings FIGS. 2A-2N, illustrate a device 10, such as a prosthesis 13, embodying features of the invention and generally including an expandable structure 16 implantable in an intracorporeal body, such as body lumen 19 including a susceptible tissue site 22, and a source 25 adjacent the expandable structure 16 including a therapeutic capable agent 28. The device 10, as shown, is disposed in the body lumen 19. It should be appreciated, that although the source 25 as depicted in the figures is disposed adjacent a surface of the expandable structure, the word adjacent is not intended to be limited by the exemplary figures or descriptions.

The expandable structure may be formed of any suitable material such as metals, polymers, or a combination thereof In one embodiment, the expandable structure may be formed of an at least partially biodegradable material, selected from the group consisting of polymeric material, metallic materials, or combinations thereof. The at least partially biodegradable material, preferably degrades over time. Examples of polymeric material include poly-L-lactic acid, having a delayed degradation to allow for the recovery of the vessel before the structure is degraded. Example of metallic material include metals or alloys degradable in the corporeal body, such as stainless steel. An exemplary stent for use in the present invention is described in US 6,602,282.

As used herein therapeutic capable agent includes at least one compound which is either therapeutic as it is introduced to the corporeal body (e.g., human subject) under treatment, or becomes therapeutic after entering the corporeal body of the subject (or exposed to the surface of the corporeal body as the case may be), by for example, reaction with a native or non-native substance or condition. Examples of native conditions include pH (e.g. acidity), chemicals, temperature, salinity, and conductivity; with non-native conditions including those such as magnetic fields, and ultrasound. In the present application, the chemical name of any of the therapeutic capable agents or other compounds is used to refer to the compound itself and to pro-drugs (precursor substances that are converted into an active form of the compound in the body), and/or pharmaceutical derivatives, analogues, or metabolites thereof (bioactive compound to which the compound converts within the body directly or upon introduction of other agents or conditions (e.g., enzymatic, chemical, energy), or environment (e.g., pH)).

The therapeutic capable agent may be selected from a group consisting of immunosuppressants, anti-inflanimatories, anti-proliferatives, anti-migratory agents, anti-fibrotic agents, proapoptotics, calcium channel blockers, anti-neoplastics, antibodies, antithrombotic agents, anti-platelet agents, IIb/IIIa agents, antiviral agents, and a combination thereof.

Specific examples of therapeutic capable agent include: mycophenolic acid, mycophenolate mofetil, mizoribine, methylprednisolone, dexamethasone, Certican^{™}, rapamycin, Triptolide^{™}, Methotrexate^{™}, Benidipine^{™}, Ascomycin^{™}, Wortmannin^{™}, LY294002, Camptothecin^{™}, Topotecan^{™}, hydroxyurea, Tacrolimus^{™} (FK 506), cyclophosphamide, cyclosporine, daclizumab, azathioprine, prednisone, Gemcitabine^{™}, derivatives and combinations thereof.

Mycophenolic acid is an immunosuppressive drug produced by the fermentation of several penicillium brevi-compactum and related species *(*The Merk Index, Tenth Edition, 1983). It has a broad spectrum of activities, specific mode of action, and is tolerable in large dose with minimal side effects, Epinette et al.,Journal of the American Academy of Dermatology, 17, pp. 962-971 (1987). Mycophenolic acid has been shown to have anti-tumor, anti-viral, anti-psoriatric, immunosuppressive, and anti-inflammatory activities, Lee et al., Pharmaceutical Research, 2, pp. 161-166 (1990), along with antibacterial and antifungal activities, Nelson et al., Journal of Medicinal Chemistry, 33, pp. 833-838 (1990). Of particular interest to the present invention, animal studies of accelerated arteriosclerosis have demonstrated that mycophenolic acid could also decrease the extent of smooth muscle cell proliferation, Gregory et al., Transplant Proc., 25, pp. 770 (1993).

Mycophenolic acid acts by inhibiting inosine monophosphate dehydrogenase and guanosine monophosphate synthetase enzymes in the de novo purine biosynthesis pathway. This may cause the cells to accumulate in the G1-S phase of the cell cycle and thus result in inhibition of DNA synthesis and cell proliferation (hyperplasia). In the present application, the term "mycophenolic acid" is used to refer to mycophenolic acid itself, pro-drugs (precursor substances that are converted into an active form of mycophenolic acid in the body), and/or pharmaceutically derivatives thereof, or metabolites thereof (bioactive compound to which the mycophenolic acid converts within the body directly or upon introduction of other agents (e.g., enzymatic, chemical, energy)). For example, a pro-drug such as mycophenolate mofetil may be biotransformed or metabolically converted to a biologically active form of mycophenolic acid when administered in the body. A number of derivatives of mycophenolic acid are taught in U.S. Patent Nos. 4,786,637, 4,753,935, 4,727,069, 4,686,234, 3,903,071, and 3,705,894 as well as pharmaceutically acceptable salts thereof.

Mizoribine acts by inhibiting inosine monophosphate dehydrogenase and guanosine monophosphate synthetase enzymes in the de novo purine biosynthesis pathway. This may cause the cells to accumulate in the G1-S phase of the cell cycle and thus result in inhibition of DNA synthesis and cell proliferation (hyperplasia).

Methylprednisolone is a synthetic steroid in the class of glucocorticoids that suppresses acute and chronic inflammations. In addition, it reduced vascular smooth muscle generation. Its anti-inflammatory actions include inhibition of accumulation of inflammatory cells (including macrophages and leukocytes) at inflammation sites, and inhibition of phagocytosis, lysosomal enzyme release, and synthesis and/or release of several chemical mediators; immunosuppressant actions may involve prevention/suppression of cell-mediated (delayed hypersensitivity) immune reactions and more specific actions affecting immune response; immunosuppressant actions may also contribute significantly to the anti-inflammatory effect.

Certican^{™}, also known as everolimus, SDZ-RAD, RAD, RAD666, or 40-0-(2-hydroxy)ethyl-rapamycin, is a potent immunosuppressant and anti-inflammatory agent. In particular, Certican^{™} acts to inhibit the activation and proliferation of T lymphocytes in response to stimulation by antigens, cytokines (IL-2, IL-4, and IL-15), and other growth-promoting lymphokines. Certican^{™} also inhibits antibody production. In cells, Certican^{™} binds to the immunophilin, FK Binding Protein-12 (FKBP-12). The Certican:FKBP-12 complex, which has no effect on calcineurin activity, binds to and inhibits the activation of the mTOR, a key regulatory kinase. This inhibition suppresses cytokine-driven T-cell proliferation, inhibiting the progression of the cell cycle from the G1 to the S phase, selectively blocking signals leading to the activation of p70s6k, p33cdk2 and p34cdc2. Thus, Certican^{™} administration results in inhibiting proliferation of T and B cells, inflammatory cells, as well as smooth muscle cells (hyperplasia).

Triptolide^{™} or related compounds, such as, tripdiolide, diterpenes, triterpenes, diterpene epoxides, diterpenoid epoxide, triepoxides, or tripterygium wifordii hook F (TWHF), are also potent immunosuppressant and anti-inflammatory agents. Specifically, Triptolide^{™} has been shown to inhibit the expression of IL-2 in activated T cells at the level of purine-box/nuclear factor and NF-kappaB mediated transcription activation. Triptolide™ may induce apoptosis in tumor cells and potentiate a tumor necrosis factor (TNF-alpFha) induction of apoptosis in part through the suppression of c-IAP2and c-IAPl induction. Triptolide^{™} inhibits the transcriptional activation, but not the DNA binding, of nuclear factor-kappaB. Triptolide^{™} may also inhibit expression of the PMA-induced genes tumor necrosis factor-alpha, IL-8, macrophage inflammatory protein- 2alpha, intercellular adhesion molecule-1, integrin beta6, vascular endothelial growth factor, granulocyte macrophage colony-stimulating factor (GM-CSF), GATA-3, fra-1, and NF45. Triptolide^{™} inhibits constitutively expressed cell cycle regulators and survival genes, such as, cyclins D1, B1, A1, cdc-25, bcl-x, and c-jun. Thus anti-inflammatory, antiproliferative, and proapoptotic properties of Triptolide^{™} are associated with inhibition of nuclear factor-kappaB signaling and inhibition of the genes known to regulate cell cycle progression and survival. Triptolide^{™} inhibits mRNA expression of c-myc and PDGF in vascular smooth muscle cells, hence resulting in the inhibition of proliferative smooth muscle cells (hyperplasia).

Methotrexate^{™}, formerly amethopterin, is an immunosuppressant and anti-proliferative agent that has been used in the treatment of certain neoplastic diseases and severe psoriasis. Chemically MethotrexateTM is N-[4[[(2,4-diamino-6-pteridinyl)methyl] methylamino]benzoyl]-L-glutamic acid. In particular, Methotrexate^{™} is a is inhibits dihydrofolic acid reductase, thereby inhibiting the reduction of dihydrofolates to tetrahydrofolates in the process of DNA synthesis, repair, and cellular replication. Actively proliferating tissues such as malignant cells, bone marrow, fetal cells, buccal and intestinal mucosa, and cells of the urinary bladder are in general more sensitive to this effect of the methotrexate. When cellular proliferation in malignant tissue is greater than in most normal tissues, methotrexate may impair malignant growth without irreversible damage to normal tissues. Approximately 50% of the drug may be reversibly bound to serum proteins. After absorption, methotrexate undergoes hepatic and intracellular metabolism to polyglutamated forms which can be converted back to methotrexate by hydrolase enzymes. These polyglutamates act as inhibitors of dihydrofolate reductase and thymidine synthetase.

Benidipine - Benidipine hydrochloride, ((±)-(R*)-3-[(R*)-1-benzyl-3-piperidyl] methyl 1,4-dihydro-2,6-dimethyl-4-(*m*-nitrophenyl)-3,5-pyridine dicarboxylate hydrochloride), is a long-acting, L-type Ca²⁺ channel blocker. Ca²⁺ channel blockers are widely used for the treatment of ischemic heart disease and systemic hypertension because of their ability to effectively dilate coronary and systemic arteries. Ca²⁺ channel blockers increase coronary blood flow (CBF) in inhibiting Ca²⁺ entry into smooth muscle cells. Since Ca²⁺ overload is deleterious for the maintenance of cellular homeostasis, Ca²⁺ channel blockers are believed to be effective in attenuating Ca²⁺ overload. Because it blocks Ca²⁺ entry, it inhibits the proliferation of smooth muscle cell.

Benidipine can protect endothelial cell function in the renal resistance arteries of hypertensive rats and the mesenteric arteries of rats subjected to circulatory shock. Endothelial cell function is important for the preservation of organ function during ischemic or hypertensive stress. Benidipine has a cardioprotective effect during myocardial ischemia and reperfusion injury. Since myocardial ischemia impairs endothelial cell function by the activation of platelets and leukocytes, benidipine may attenuate endothelial cell dysfunction and increase the production of nitric oxide in ischemic hearts.

Ascomycin (molecular formula: C₄₃H₆₉NO₁₂; molecular weight: 792.02; CAS No. 104987-12-4) has produced significant anti-inflammatory and immunosuppressant activity. Ascomycin has been shown to selectively inhibit inflammatory cytokine release. The drug binds to the cytosolic immunophilin receptor macrophilin-12, and the resulting complex inhibits the phosphatase calcineurin, thus blocking T-cell activation and cytokine release. It inhibits production of Th1 cytokines (interleukin-2 and interferon-gamma) and Th2 cytokines (interleukin-10 and interleukin-4). Ascomycin has also been demonstrated to similarly inhibit mast cell. Strong immunosuppressant; inhibits allogenic T-lymphocyte proliferation. It binds with high affinity to FKBP and inhibits calcineurin phosphatase in the nM range.

Ascomycin affects calcineurin-mediated signal transduction. It is a natural product of bacteria and fungi, respectively, with potent immunosuppressive, anti-inflammatory, and antimicrobial activity. Despite differing chemical structures, ascomycin is a macrolide where its mechanisms of action and cellular effects results in the inhibition of the protein phosphatase calcineurin. This drug is hydrophobic and thought to diffuse across the plasma membrane; once inside the cell, Ascomycin forms complexes with their major receptors, FKBP12. FKBP12 is small, ubiquitous, cytosolic proteins that catalyse *cis-trans* prolyl isomerization, a reaction that can be a rate-limiting step in protein folding. Binding of ascomycin to FKBP12 inhibits prolyl-isomerase activity. However, this inhibition is not the major toxic effect in the cell; instead the FKBP12-ascomycin complex binds to and inhibit calcineurin (a serine-threonine-specific protein phosphatase), which is activated by calmodulin in response to intracellular calcium-ion increases. The molecular nature of this interaction is now known in considerable detail, as the structures of both calcineurin alone and in a ternary complex with FKBP12-ascomycin have both been solved at high resolution.

Wortmannin (CAS No. 19545-26-7, synonym SL-2052, molecular formula: C23H24O8 formula weight: 428.4 (anhydrous)) has significant anti-inflammatory and immunosuppressant activity. Wortmannin, a fungal metabolite, is a specific and potent inhibitor of myosin light chain kinase and a potent inhibitor of neutrophil activation by inhibiting F-met-leu(FMLP)-phe-stimulated superoxide anion production without affecting intracellular calcium mobilization. It inhibits FMLP-stimulated phospholipase D activation without direct inhibition of the enzyme. It also inhibits phosphatidylinositol-3-kinase (PI3-kinase) and blocks IgE-mediated histamine release in rat basophilic leukemia cells and human basophils.

Wortmannin is a potent and specific inhibitor of phosphatidylinositol 3-kinase (P13-K) with an IC₅₀ of 2-4 nM; and inhibits myosin light chain kinase at a 100-fold higher concentration. Inhibition of PI3-K/Akt signal transduction cascade enhances the apoptotic effects of radiation or serum withdrawal and blocks the antiapoptotic effect of cytokines. Inhibition of PI3-K by wortmannin also blocks many of the short-term metabolic effects induced by insulin receptor activation.

Phosphatidylinositol-3-kinase participates in the signal transduction pathway responsible for histamine secretion following stimulation of high affinity immunoglobulin E receptor (FceRI). Wortmannin blocks these responses through direct interaction with the catalytic subunits (110 kDa) of PI3-kinase enzyme. Wortmannin inhibited the activity of partially purified PI3-kinase from calf thymus at concentrations as low as 1.0 nM and with IC50 values of 3.0 nM. Inhibition was irreversible. Wortmannin inhibited both FceRI-mediated histamine secretion and leukotriene release up to 80% with IC50 values of 2.0 and 3.0 nM, respectively. Additional functions of Wortmannin follows: immunosuppressive activity, strong anti-inflammatory activity, suppression of cellular responses such as respiratory burst and exocytosis in neutrophils and catecholamine release in adrenal chromaffin cells. Aggregation and serotonin release in platelets were reported using a final concentration of 1 M of wortmannin in 0.01% DMSO.

Wortmannin is a hydrophobic steroid-related product of the fungus *Talaromyces wortmanni* that inhibits signal-transduction pathways; for example, wortmannin inhibits stimulation of neutrophils, histamine secretion by basophilic leukaemia cells and nitric-oxide production in chicken macrophages . In mammalian cells, several lines of evidence indicate that the growth-factor-activated PI-3 kinase is potently inhibited by wortmannin. First, wortmannin blocks the antigen-dependent stimulation of PI-3-kinase activity in basophils 54 and the insulin-stimulated PI-3-kinase activity in adipocytes. Wortmannin also inhibits stimulated PIns-(3,4,5)P 3 production in neutrophils, consistent with a block in PIns-(4,5)P phosphorylation by PI-3 kinase; purified p110-p85 PI-3 kinase is potently inhibited by wortmannin *in vitro.* Finally, studies with anti-wortmannin antibodies and site-directed mutagenesis reveal that wortmannin forms a covalent complex with an active-site residue of bovine PI-3 kinase, lysine 802 of the 110 kDa catalytic subunit. This active-site lysine residue is essential for PI-3 kinase activity and is well conserved throughout all members of the PI-kinase-related protein family.

LY294002 has produced significant anti-inflammatory and immunosuppressant activity. LY294002 has been used in some cases to confirm the effects of wortmannin attributed to inhibition of PI-3 kinase, but this compound also inhibits mTOR and may inhibit other wortmannin targets as well. Hence, more enzyme-specific analogues of wortmannin would be valuable reagents to probe the intracellular functions of this intriguing family of enzymes. The wortmannin analogue demethoxyviridin has been shown to inhibit an as-yet-unidentified PI-4-kinase activity in *Schizosaccharomyces pombe* that is much less sensitive to wortmannin, indicating that analogues with greater specificity may be obtained.

Camptothecin and Topotecan (Hycamtin) - Camptothecin (molecular formula: C₂₀H₁₆N₂O₄, molecular weight: 348.4, CAS No. 7689-03-4) and its analogues, including topotecan (9-Dimethylaminomethyl-10-hydroxycamptothecin, HCI salt 1H-Pyrano[3',4':6,7]indolizino[1,2-b]quinoline-3,14(4H,12H)- dione, 4-ethyl-4,9-dihydroxy-10-[(dimethylamino)methyl]-,HCI salt (S) molecular formula: C₂₃H₂₃N₃O₅ . HC1, molecular weight: 457.9), are anti-neoplastic agents, believed to exert cytotoxic effects through the inhibition of topoisomerase I. This is the only known class of drug that exhibits this mechanism of action. However, inhibition of topoisomerase activity is not an unknown mechanism of action since many classes of drugs *(eg. epipodophyllotoxins)* operate through inhibition of topoisomerase II *(topo II).*

Topoisomerases are enzymes which break strands of DNA so that the strands can be rotated around each other and then the break resealed. They can be divided into two classes according to the nature of the mechanisms of action they employ.

Type I topoisomerase is a monomeric protein of about 100 Kilodaltons *(KDa).* It is capable of making a transient break in a single strand of the DNA helix. This reduces the torsional strain on the DNA and allows the DNA to unwind ahead of the replication fork. This enzyme is capable of relaxing highly negatively supercoiled DNA. In the eukaryotic version of this enzyme, a phosphotyrosyl bond is formed between the enzyme and the 3' end of the DNA break. In this process there is a transfer of a phosphodiester bond in the DNA to the protein. The structure of the DNA is manipulated and the DNA is rejoined. Since the reaction requires only the transfer of bonds, not irreversible hydrolysis, no input of energy is required. Topo I is believed to function in DNA replication, RNA transcription, genetic recombination, chromosomal condensation/decondensation and in viral encapsulation. Its presence is not cell-cycle dependent and it is found in quiescent as well as proliferating cells. It appears, however, that this enzyme is not required for the viability of cells. Topo II seems to fulfill the functions of topo I when it is absent. Double mutants, which lack both topo I and II have defects of replication and transcription.

Cells lacking the topo I enzyme are resistant to camptothecin, while cells containing higher topo I levels are hypersensitive to these drugs. The camptothecins appear to block the rejoining step of the breakage-reunion reaction of the enzyme, leaving the enzyme covalently bound to DNA. This results in protein associated single strand breaks in the DNA.

Topotecan has demonstrated good antitumor activity *(increased life spans (ILS) > 95%s)* in several intraperitoneally (IP) and intravenously *(IV)* implanted murine tumor systems, including P388 leukemia, L1210 leukemia, B16 melanoma, Lewis lung carcinoma and M5076 reticulum cell sarcoma. Topotecan was equally effective when administered IP or IV against IP or IV implanted tumors. Subcutaneous administration did not result in any local tissue damage. This drug was also equally effective when administered enterally or parenterally in some tumors, suggesting that, in mice, the bioavailability is high.

The antitumor activity of topotecan in tumor-bearing mice can be enhanced by using an intermittent dosing regimen. Results were dependent upon how sensitive the tumor model was to bolus treatment with topotecan. In studies in which topotecan was administered every three hours for 4 doses, a broader therapeutic dose range was noted in tumors that were quite sensitive to bolus therapy, including IV-implanted L1210 leukemia, IP M5076 reticulum sarcoma, SC colon 51, and SC B16 melanoma. In tumor types that were less sensitive to bolus therapy, such as SC implanted colon 26 and Madison 109 lung carcinomas, the divided dose resulted in a greater degree of inhibition at the MTD.

The activity of topotecan has also been investigated using a human tumor clonogenic assay. Fifty-five human tumor specimens were exposed to topotecan for one hour at a concentration of either 1 of 10 ug/ml or as a continuous exposure *(0.1 or 1.0 ug*/*ml).* At a concentration of 0.1 ug/ml of continuous exposure, response rates of 29, 27, and 37% were seen against breast, nonsmall cell lung, and ovarian cancers, respectively, Activity was also seen against stomach, colon, and renal cancer, and mesothelioma. Incomplete cross-resistance was noted with doxorubicin, 5-FU and cyclophosphamide.

One of the most promising new drug classes includes the topoisomerase I inhibitors. This class is structurally related to the natural compound camptothecin, which is derived from the Chinese Camptotheca acuminata plant. Topoisomerase I inhibitors differ from topoisomerase II inhibitors, such as etoposide, in that they bind to the topoisomerase-DNA complex; cell death ensues when the DNA helix cannot rebuild after uncoiling. The two most promising compounds in this class are irinotecan and topotecan; in Phase II trials, they have shown activity against a variety of cancers, including colorectal cancer. The success of topotecan in patients with previously treated small-cell lung cancer (response rate of as high as 39 percent) and ovarian cancer (response rate as high as 61 percent) has increased interest in Phase III trials with this drug.

Hydroxyurea (Hydrea) - Hydroxyurea (molecular formula: **CH₄N₂O₂,** molecular weight: 76.06, CAS No. 127-07-1) is an Antineoplastic Agent. It is readily available drug that has been in use for three decades in treating certain kinds of leukemia and other cancers; it may also be promising for treatment of sickle cell disease. The exact mechanism of action has been unknown. It has been known that hydroxyurea immediately inhibits DNA synthesis without inhibiting the synthesis of RNA or protein, but until recently it was not known how it did this.

Gemcitabine (Gemzar) (Gemcitabine hydrochloride; 2'-deoxy-2',2'-difluorocytidine) is an Antineoplastic Agent. Gemcitabine induces programmed cell death and activates protein kinase C in BG-1 human ovarian cancer cells. It is a known antitumor nucleoside where the mechanism of action of gemcitabine is via inhibition of DNA and RNA synthesis.

Gemcitabine is a novel deoxycytidine analogue, a pyrimidine antimetabolite related to cytarabine, which was originally investigated for its antiviral effects but has since been developed as an anticancer therapy. Gemcitabine exhibits cell phase specificity, primarily killing cells undergoing DNA synthesis (S-phase) and also blocking the progression of cells through the G1/S-phase boundary. Gemcitabine is a pro-drug and is metabolized intracellularly to the active diphosphate (dFdCDP) and triphosphate (dFdCTP) nucleosides. The cytotoxic effects of gemcitabine are exerted through dFdCDP-assisted incorporation of dFdCTP into DNA, resulting in inhibition of DNA synthesis and induction of apoptosis.

Gemcitabine exhibits significant cytotoxicity activity against a variety of cultured murine and human tumor cells.It exhibits cell phase specificity, primarily killing cells undergoing DNA synthesis (S-phase) and under certain conditions blocking the progression of cells through the G 1/S-phase boundary.In vitro the cytotoxic action of gemcitabine is both concentration and time dependant.

In animal tumor models, the antitumor activity of gemcitabine is schedule dependant. When administered daily gemcitabine causes death in animals with minimal anti-tumor activity. However when every 3rd or 4th day dosing schedule is used, gemcitabine can be given at non-lethal doses that have excellent anti-tumor activity against a broad range of mouse tumors.

In an embodiment, the source of the therapeutic capable agent is a polymeric material including therapeutic capable agent moieties as a structural subunit of the polymer. The therapeutic capable agent moieties are polymerized and associated to one another through suitable linkages (e.g. ethylenic) forming polymeric therapeutic capable agent. Once the polymeric therapeutic capable agent is brought into contact with tissue or fluid such as blood, the polymeric therapeutic capable agent subunits disassociate. Alternatively, the therapeutic capable agent may be released as the polymeric therapeutic capable agent degrades or hydrolyzes, preferably, through surface degradation or hydrolysis, making the therapeutic capable agent available to the susceptible tissue site, preferably over a period of time. Examples of methods and compounds for polymerizing therapeutic capable agents are described in WO 99/12990 Patent Application by Kathryn Uhrich, entitled "Polyanhydrides With Therapeutically Useful Degradation Products," and assigned to Rutgers University. An example of a therapeutic capable agents and a suitable reaction ingredient unit includes, mycophenolic acid with adipic acid and/or salicylic acid in acid catalyzed esterification reaction; mycophenolic acid with aspirin and/or adipic acid in acid catalyzed esterification reaction, mycophenolic acid with other NSAIDS, and/or adipic acid in acid catalyzed esterification reaction. In an embodiment, the polymeric therapeutic capable agent may be associated with a polymeric and/or metallic backbone.

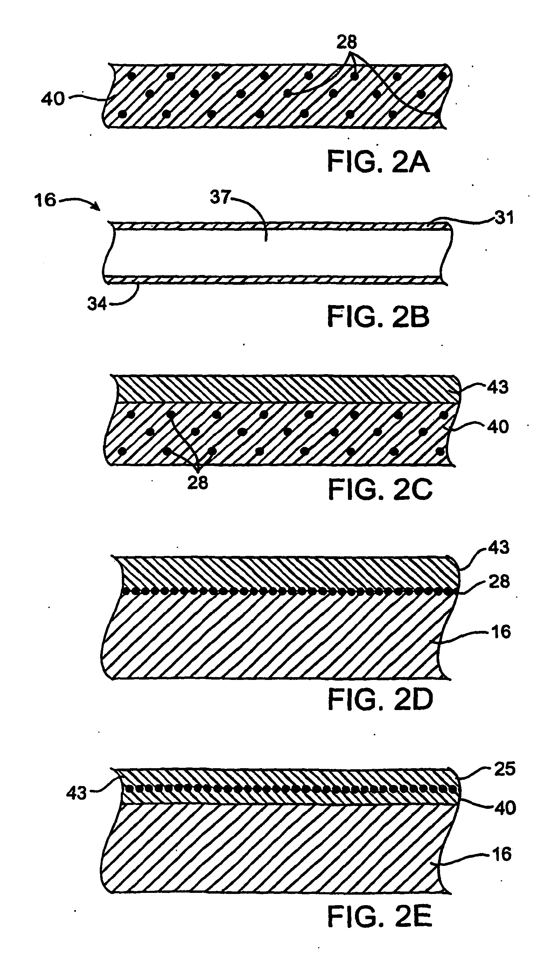

The expandable structure 16, as shown without intending any limitation, has a tissue facing surface 31 and luminal facing surface 34, and optionally an interior 37 which may include a lumen as shown in FIG. 2B. It will be appreciated that the following depictions are for illustration purposes only and do not necessarily reflect the actual shape, size, configuration, or distribution of the prosthesis 13. The prosthesis may have a continuous structure or an intermittent structure as the case may be with many stents (e.g., the cross section of the stent does not entirely include a substrate forming the expandable structure - for example, some stents have a screen or mesh like cross section). The source may be disposed or formed adjacent at least a portion of either or both the luminal facing surface, as shown in FIG. 1B; and the tissue facing surface, as shown in FIG. 1C; within the interior of the expandable structure, or any combination thereof.

The source 25 for making the therapeutic capable agent available to therapeutic capable agent is associated with expandable structure, in one or more configurations. The source as shown in FIGS. 2A and 2B is within the expandable structure 16, as for example, when a matrix 40 is formed by the expandable structure 16 and the therapeutic capable agent 28, or when the therapeutic capable agent 28 is disposed within the interior (or the exterior of the expandable structure 16 as the case may be), 37 of the expandable structure 16.

Now referring to FIG. 2C, the source may further comprises a rate-controlling element 43, may be formed over at least a portion of the expandable structure 16 for controlling the release of the therapeutic capable agent 28 from the matrix 40 or the interior 37 of the expandable structure. By way of example, the source may be the rate-controlling element itself when the therapeutic capable agent is a polymeric therapeutic capable agent.

The rate-controlling element may be formed of a non-degradable, partially degradable, substantially degradable material, or a combination thereof. The material may be synthetic or natural; non-polymeric, polymeric or metallic; or a combination thereof. By way of examples, a metallic material that at least partially degrades with time may be used as the rate-controlling element; as well as non-polymers having large molecular weight, polar or non-polar functional groups, electrical charge, steric hindrance groups, hydrophobic, hydrophilic, or amphiphilic moieties.

Suitable biodegradable rate-controlling element materials include, but are not limited to, poly(lactic acid), poly(glycolic acid) and copolymers, poly dioxanone, poly (ethyl glutamate), poly (hydroxybutyrate), polyhydroxyvalerate and copolymers, polycaprolactone, polyanhydride, poly(ortho esters); poly (iminocarbonates), polycyanoacrylates, polyphosphazenes, copolymers and other aliphatic polyesters, or suitable copolymers thereof including copolymers of poly-L-lactic acid and poly-e-caprolactone; mixtures, copolymers, and combinations thereof.

Suitable nondegradable or slow degrading rate-controlling element materials include, but are not limited to, polyurethane, polyethylenes imine, cellulose acetate butyrate, ethylene vinyl alcohol copolymer, silicone, polytetrafluorethylene (PTFE), parylene, parylast, poly (methyl methacrylate butyrate), poly-N-butyl methacrylate, poly (methyl methacrylate), poly 2-hydroxy ethyl methacrylate, poly ethylene glycol methacrylates, poly vinyl chloride, poly(dimethyl siloxane), poly(tetrafluoroethylene), poly (ethylene oxide), poly ethylene vinyl acetate, poly carbonate, poly acrylamide gels, N-vinyl-2-pyrrolidone, maleic anhydride, Nylon, cellulose acetate butyrate (CAB) and the like, including other synthetic or natural polymeric substances; mixtures, copolymers, and combinations thereof. In an embodiment the rate-controlling element is formed from a material selected from the group consisting of silicone, polytetrafluoroethylene, parylast, polyurethane, parylene, cellulose acetate butyrate; mixtures, copolymers and combinations thereof.

Suitable natural material include: fibrin, albumin, collagen, gelatin, glycosoaminoglycans, oligosaccharides & poly saccharides, chondroitin, phosholipids, phosphorylcholine, glycolipids, proteins, amino acids, cellulose, and mixtures, copolymers, or combinations thereof. Other suitable material include, titanium, chromium, Nitinol, gold, stainless steel, metal alloys, or a combination thereof; and other compounds that may release the therapeutic capable agent as a result of interaction (e.g., chemical reaction, high molecular weight, steric hindrance, hyrophobicity, hydrophilicity, amphilicity, heat) of the therapeutic capable agent with the rate-controlling element material (e.g, a non-polymer compound). By way of example, a combination of two or more metals or metal alloys with different galvanic potentials to accelerate corrosion by galvanic corrosion pathways may also be used.

In another embodiment, the surface of the structure may be pre-processed using any of a variety of procedures, including, cleaning; physical modifications such as etching or abrasion; and chemical modifications such as solvent treatment, the application of primer coatings, the application of surfactants, plasma treatment, ion bombardment, and covalent bonding. In an embodiment, a metal film or alloy with a small pits or pin holes to accelerate corrosion by pitting corrosion, allowing the pin hole formed by the corrosion to act as an orifice for drug release. In an embodiment, the therapeutic capable agent may be attached to the metal or metal alloy.

An example of a biodegradable material of the present invention is a copolymer of poly-L-lactic acid (having an average molecular weight of about 200,000 daltons) and poly-e-caprolactone (having an average molecular weight of about 30,000 daltons). Poly-e-caprolactone (PCL) is a semi crystalline polymer with a melting point in a range from 59 °C to 64 °C and a degradation time of about 2 years. Thus, poly-1-lactic acid (PLLA) can be combined with PCL to form a matrix that generates the desired release rates. A preferred ratio of PLLA to PCL is 75:25 (PLLA/PCL). As generally described by Rajasubramanian et al. in ASAIO Journal, 40, pp. M584-589 (1994), the full disclosure of which is incorporated herein by reference, a 75:25 PLLA/PCL copolymer blend exhibits sufficient strength and tensile properties to allow for easier coating of the PLLA/PLA matrix on the expandable structure. Additionally, a 75:25 PLLA/PCL copolymer matrix allows for controlled drug delivery over a predetermined time period as a lower PCL content makes the copolymer blend less hydrophobic while a higher PLLA content leads to reduced bulk porosity.

The degradable material may degrade by bulk degradation or hydrolysis. In an embodiment, the rate-controlling element degrades or hydrolyzes throughout, or preferably, by surface degradation or hydrolysis, in which a surface of the rate-controlling element degrades or hydrolyzes over time while maintaining bulk integrity. In another embodiment, hydrophobic rate-controlling elements are preferred as they tend to release therapeutic capable agent at desired release rate. A non-degradable rate-controlling element may release therapeutic capable agent by diffusion. By way of example, if the rate-controlling element is formed of non-polymeric material, the therapeutic capable agent may be released as a result of the interaction (e.g., chemical reaction, steric hinderence, hyrophobicity, hydrophilicity, amphilicity) of the therapeutic capable agent with the rate-controlling element material (e.g, a non-polymer compound). In an embodiment, when the rate-controlling element does not form, at least a sufficient matrix with the therapeutic capable agent, the therapeutic capable agent may be released by diffusion through the rate-controlling element.

By way of example, a rate-controlling element having low molecular weight and/or relatively high hydrophilicity in the tissue or blood, may diffuse through the source (e.g., a matrix), thus, increasing the surface area or volume for the therapeutic capable agent to be released from, thus, affecting the release rate of the therapeutic capable agent.

In yet another embodiment the therapeutic capable agent is made available to the susceptible tissue site as the native environment of the area where the device is implanted changes. For example, a change in the pH of the area where the device is implanted may change over time so as to bring about the release of the therapeutic capable agent directly (as for example when a polymeric drug acts as the matrix including both the therapeutic capable agent and the rate-controlling element), or indirectly by affecting the erosion or diffusion characteristic of the rate-controlling element as either or both the matrix or non-matrix. For example, as the pH increases or decreases, the erosion of the rate-controlling element changes allowing for initial and subsequent phase releases.

FIG. 2D illustrates features of an embodiment having the therapeutic capable agent 28 disposed between one of the tissue or luminal facing surfaces of the expandable structure and the rate-controlling element 43.

As shown in FIG. 2E, the source 25 includes the rate-controlling element 43 formed adjacent at least a portion of one of the tissue or luminal facing surfaces of the expandable structure 16 and forming the matrix 40 with the therapeutic capable agent 28. As noted earlier, the therapeutic capable agent 28 may itself act as a rate-controlling element, as for example, when the polymeric therapeutic capable agent forms a matrix.

The matrix may be formed between the rate-controlling element 43 and the expandable structure 16 and forming a matrix interface 46 therebetween and/or between the therapeutic capable agent 28 and the rate-controlling element 43, as shown in FIGS. 2F and 2G.

In an embodiment, features of which are shown in FIG. 2H, the outer most layer of the prosthesis 13 may be formed of the therapeutic capable agent with or without a matrix interface 46 formed between the outer most layer and the other layers. It should be noted, that the therapeutic capable agent 28, although as shown in most figures as discrete particles, may form a smooth layer or a layer of particles, as for example as part of matrix interface 46 as shown in FIG. 2H.

In an alternate embodiment, features of which are shown in FIG. 2I, at least one layer of a second rate-controlling element 49 is formed over the matrix 40, further affecting the release rate of the therapeutic capable agent 28 to the susceptible tissue site. The second rate-controlling element 49 may be of the same or different material than that forming the first rate-controlling element 43.

Now referring now to FIGS. 2J and 2K, the source may comprise, a plurality of compounds, as for example the first therapeutic capable agent 28 and another compound 50 such as another therapeutic capable agent 50 or an enabling compound 61 (FIG. 2N). Each of the plurality of compounds may be in the same or different area of the source. For example, as shown in FIG. 2J, the first therapeutic capable agent 28 may be present in matrix 40 while the second therapeutic capable agent 50 is in a second matrix 52 formed by the second therapeutic capable agent 50 and a second rate-controlling element 55. The rate-controlling elements 43 and 55 may be formed from the same or different material.

The another therapeutic capable agent may act in synergy with the therapeutic capable agent, in ways such as compensating for the possible reactions and by-products that can be generated by the therapeutic capable agent. By way of example, the therapeutic capable agent may reduce generation of desired endothelial cells, thus by including a suitable another therapeutic capable agent, more endothelialization may be achieved.

The another therapeutic capable agent may comprise at least one compound selected from the group consisting of anti-cancer agents; chemotherapeutic agents; thrombolytics; vasodilators; antimicrobials or antibiotics antimitotics; growth factor antagonists; free radical scavengers; biologic agents; radiotherapeutic agents; radiopaque agents; radiolabelled agents; anti-coagulants such as heparin and its derivatives; anti-angiogenesis drugs such as Thalidomide^{™}; angiogenesis drugs; PDGF-B and/or EGF inhibitors; anti-inflamatories including psoriasis drugs; riboflavin; tiazofurin; zafurin; anti-platelet agents including cyclooxygenase inhibitors such as acetylsalicylic acid, ADP inhibitors such as clopidogrel (e.g., Plavix^{™}) and ticlopdipine (e.g., ticlid^{™}), phosphodiesterase III inhibitors such as cilostazol (e.g., Pletal™), glycoprotein IIb/IIIa agents such as abciximab (e.g., Rheopro™); eptifibatide (e.g., Integrilin™), and adenosine reuptake inhibitors such as dipyridmoles; healing and/or promoting agents including anti-oxidants, nitrogen oxide donors; antiemetics; antinauseants; derivatives and combinations thereof.

The another therapeutic agent may be released prior to, concurrent with, or subsequent to, the therapeutic capable agent, at similar or different rates and phases.

In another embodiment, features of which are shown in FIGS. 2L and 2M, the therapeutic capable agent 28 is disposed within or on the expandable structure 16 within a reservoir 58. The rate-controlling element 43 may be disposed adjacent the reservoir 58 and/or the therapeutic capable agent 28 for affecting the release of the therapeutic capable agent. As stated earlier, the exemplary figures and descriptions are not meant to limit the term "adjacent."

In a further embodiment, features of which are shown in FIG. 2N, the another compound comprises the enabling compound 61 respondable to an external form of energy, or native condition, to affect the release of the therapeutic capable agent. The respondable compound may be associated with the therapeutic capable agent, the rate-controlling element, the expandable structure, or a combination thereof. As shown in FIG. 2N, the respondable compound is associated with the therapeutic capable agent. The enabling compound 61 may be formed from magnetic particles coupled to the therapeutic capable agent 28. The energy source may be a magnetic source for directing a magnetic field at the prosthesis 13 after implantation to effect release of the therapeutic capable agent 28. The magnetic particles 61 may be formed from magnetic beads and will typically have a size in a range from about 1 nm to about 100 nm. The magnetic source exposes the prosthesis 13 to its magnetic field at an intensity typically in the range from about O.O1T to about 2T, which will activate the magnetic particles 61 and thereby effect release of the therapeutic capable from the prosthesis. The another enabling compound may be present in other configurations of prosthesis 13 as described above.

Other suitable external energy sources, which may or may not require a second compound or their performance may not be affected by the presence or absence of a second compound, include ultrasound, magnetic resonance imaging, magnetic field, radio frequency, temperature change, electromagnetic, x-ray, radiation, heat, gamma, vibration, microwave, or a combination thereof.

By way of example, an ultrasound external energy source may be used having a frequency in a range from 20 kHz to 100 MHz, preferably in a range from 0.1 MHz to 20 MHz, and an intensity level in a range from 0.05 W/cm² to 10 W/cm², preferably in a range from 0.5 W/cm² to 5 W/cm². The ultrasound energy would be directed at the prosthesis 13 from a distance in a range from 1 mm to 30 cm, preferably in a range from 1 cm to 20 cm. The ultrasound may be continuously applied or pulsed, for a time period in a range from 5 sec to 30 minutes, preferably in a range from 1 minute to 15 minutes. The temperature of the prosthesis 13 during this period will be in a range from 36°C to 48°C. The ultrasound may be used to increase a porosity of the prosthesis 13, thereby allowing release of the therapeutic capable agent 28 from the prosthesis 13. Other sources of energy, for example, heat or vibrational, may also be used to increase the porosity of the prosthesis or a portion thereof or alter the configuration of the same.

Furthermore, a biocompatible (e.g., blood compatible) layer may be formed over the source and/or the most outer layer of the device, to make or enhance the biocompatibility of the device. Suitable biocompatible material for use as the biocompatible layer include, but are not limited to, polyethylene glycol (PEG), polyethylene oxide (PEO), hydrogels, silicone, polyurethanes, heparin coatings.

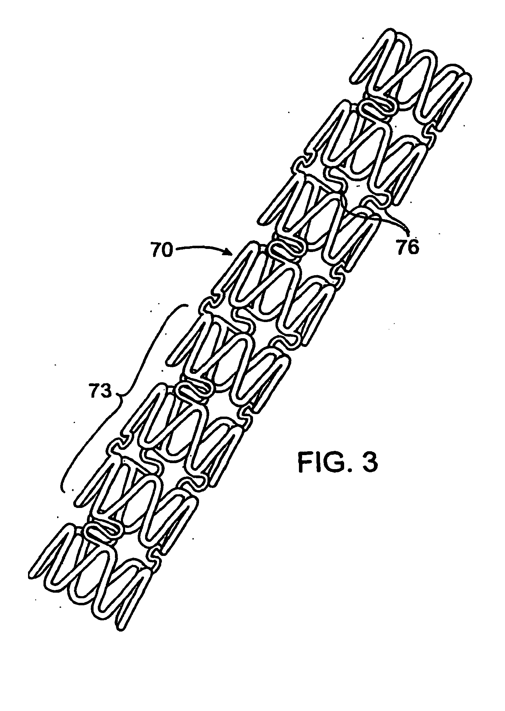

The expandable structure 16 may be a stent 70 or, a graft. When the expandable structure is a stent, the expandable structure 16 will usually comprise at least two radially expandable, usually cylindrical, ring segments 73 as shown in FIG. 3. Typically, the expandable structure 16 will have at least four, and often five, six, seven, eight, ten, or more ring segments. At least some of the ring segments will be adjacent to each other but others may be separated by other non-ring structures. The description of exemplary stent structures are not intended to be exhaustive, and it should be appreciate that other variations of stent designs usable in the present invention are known to those skilled in the art.

Referring back to FIG. 3, an exemplary stent 70 (embodying features of a stent described in more detail in U.S. Patent No. 6,602,281). for use in the present invention comprises from 4 to 50 ring segments 73 (with seven being illustrated). Each ring segment 73 is joined to the adjacent ring segment by at least one of sigmoidal links 76 (with three being illustrated). Each ring segment 73 on each ring segment 73 will be joined by the sigmoidal links76 to the adjacent ring segment. Stent 70 as shown in FIG. 3 shows the stent 70 is in a collapsed or non-expanded configuration.

The term "radially expandable" as used herein includes segments that can be converted from a small diameter configuration to a radially expanded, usually cylindrical, configuration which is achieved when the expandable structure 16 is implanted at a desired target site. The expandable structure 16 may be minimally resilient, e.g., malleable, thus requiring the application of an internal force to expand and set it at the target site. Typically, the expansive force can be provided by a balloon, such as the balloon of an angioplasty catheter for vascular procedures. The expandable structure 16 preferably provides sigmoidal links between successive unit segments which are particularly useful to enhance flexibility and crimpability of the stent.

Alternatively, the expandable structure 16 can be self-expanding. Structures for use in the devices of the present invention, including the expandable structure 16 (such as self-expanding structures) are provided by utilizing a resilient material, such as a tempered stainless steel, or a superelastic alloy such as a Nitinol^{™} alloy, and forming the body segment so that it possesses its desired, radially-expanded diameter when it is unconstrained, i.e. released from the radially constraining forces of a sheath. In order to remain anchored in the body lumen, the expandable structure 16 will remain partially constrained by the lumen. The self-expanding expandable structure 16 can be tracked and delivered in its radially constrained configuration, e.g., by placing the expandable structure 16 within a delivery sheath or tube and removing the sheath at the target site.

The dimensions of the expandable structure will depend on its intended use. Typically, the expandable structure will have a length in a range from about 5 mm to about 100 mm, usually being from about 8 mm to about 50 mm, for vascular applications. The diameter of a cylindrically shaped expandable structure for vascular applications, in a non-expanded configuration, usually ranges from about 0.5 mm to about 10 mm, more usually from about 0.8 mm to about 8 mm; with the diameter in an expanded configuration ranging from about 1.0 mm to about 100 mm, preferably from about 2.0 mm to about 30 mm. The expandable structure usually will have a thickness in a range from about 0.025 mm to 2.0 mm, preferably from about 0.05 mm to about 0.5 mm.

The ring segments, and other components of structures such as the expandable structure 16, may be formed from conventional materials used for body lumen stents and grafts, typically being formed from malleable metals or alloyes, such as 300 series stainless steel, or from resilient metals, such as superelastic and shape memory alloys, e.g., Nitinol^{™} alloys, spring stainless steels, and the like; non-metallic materials, such as polymeric materials, or a combination thereof. The polymeric materials may include those polymeric materials that are substantially non-degradable, such as those described in relation to the materials of choice for the rate-controlling element. Alternatively, the polymeric material may be a biodegradable or substantially biodegradable polymer such as those described in reference with the biodegradable rate-controlling element material. When the expandable structure material is formed of the rate-controlling element material, the expandable structure may function both as the prosthesis and the direct source of the therapeutic capable agent. Additional structures for the body or unit segments of the present invention are illustrated in U.S. Patent Nos. 5,195,417; 5,102,417; and 4,776,337.
Other suitable material for use as the structure include, carbon or carbon fiber, cellulose acetate, cellulose nitrate, silicone, polyethylene terphthalate, polyurethane, polyamide, polyester, polyorthoester, polyanhydride, polyether sulfone, polycarbonate, polytetrafluoroethylene, or another biocompatible polymeric materials, or mixtures or copolymers thereof, a polyanhydride, polycaprolactone, polyhydroxybutyrate valerate or another biodegradable polymer, or mixtures or copolymers thereof; a protein, an extracellular matrix component, collagen, fibrin or another biologic agent, or a suitable mixture of any of the material listed above, degradable, non-degradalbe, metallic, or otherwise. In an embodiment, device may comprise a biodegradable structure with a polymeric source, such as a polymeric therapeutic capable agent.

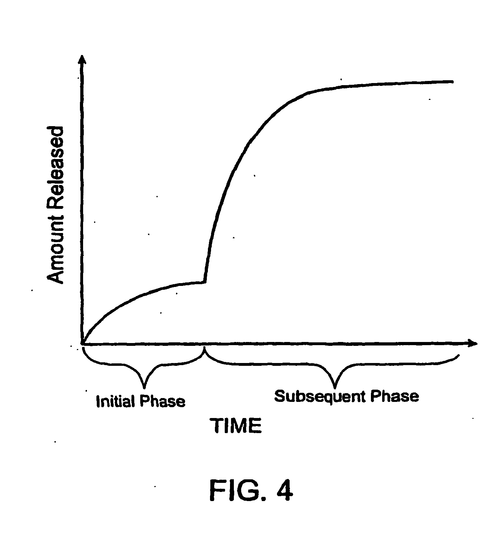

Referring now to FIG. 4, a graphical representation of an exemplary embodiment of therapeutic capable agent release over a predetermined time period is shown. The predetermined rate pattern shown in FIG. 4 of the present invention improves the efficacy of the delivery of the therapeutic capable agent to the susceptible tissue site by making the therapeutic capable agent available at none to some lower delivery rate during an initial phase. Once a subsequent phase is reached, the delivery rate of the therapeutic capable agent may be substantially higher. Thus, time delayed therapeutic capable agent release can be programmed to impact restenosis (or other targeted conditions as the case may be) at at least a partial formation of the initial cellular deposition or proliferation (hyperplasia). The present invention can further reduce the washout of the therapeutic capable agent by timing the release of the therapeutic capable agent to occur after at least initial cellularization. Moreover, the predetermined rate pattern may reduce the loading and/or concentration of the therapeutic capable agent. The predetermined rate pattern may further provide limited or reduced to no hindrance to endothelialization of the vessel wall due to the minimization of washout of the therapeutic capable agent and the increased efficiency of its release.

The devices of the present invention may be configured to release or make available the therapeutic capable agent at one or more phases, the one or more phases having similar or different performance (e.g., release) profiles. The therapeutic capable agent may be made available to the tissue at amounts which may be sustainable, intermittent, or continuous; in one or more phases and/or rates of delivery; effective to reduce any one or more of smooth muscle cell proliferation, inflammation, immune response, hypertension, or those complementing the activation of the same. Any one of the at least one therapeutic capable agents may perform one or more functions, including preventing or reducing proliferative/restenotic activity, reducing or inhibiting thrombus formation, reducing or inhibiting platelet activation, reducing or preventing vasospasm, or the like.

The total amount of therapeutic capable agent made available to the tissue depends in part on the level and amount of desired therapeutic result. The therapeutic capable agent may be made available at one or more phases, each phase having similar or different release rate and duration as the other phases. The release rate may be pre-defined. In an embodiment, the rate of release may provide a sustainable level of therapeutic capable agent to the susceptible tissue site. In another embodiment, the rate of release is substantially constant. The rate may decrease and/or increase over time, and it may optionally include a substantially non-release period. The release rate may comprise a plurality of rates. In an embodiment the plurality of release rates include at least two rates selected from the group consisting of substantially constant, decreasing, increasing, substantially non-releasing.

The total amount of therapeutic capable agent made available or released will typically be in an amount ranging from about 0.1 ug to about 10 g; generally from about 0.1 ug to about 10 mg, preferably from about 1 ug to about 10 mg, more preferably from about 1 ug to about 2 mg, from 10 ug to about 2 mg, or from about 50 ug to about 1 mg.

In an embodiment, the therapeutic capable agent may be released in a time period, as measured from the time of implanting of the device, ranging from about 1 day to about 200 days; from about 1 day to about 45 days; or from about 7 days to about 21 days.

In an embodiment the release rate of the therapeutic capable agent per day may range from about 0.001 micrograms (ug) to about 200 ug, preferably, from about 0.5 ug to about 200 ug, and most preferably, from about 1 ug to about 60 ug.

The therapeutic capable agent may be made available at an initial phase and one or more subsequent phases. When the therapeutic capable agent is delivered at different phases, the initial delivery rate will typically be from about 0 to about 99 % of the subsequent release rates, usually from about 0 % to about 90 %, preferably from about 0 % to 75 %. In an embodiment a mammalian tissue concentration of the substance at an initial phase will typically be within a range from about 0.001 nanogram (ng)/mg of tissue to about 100 ug/mg of tissue; from about 1 ng/mg of tissue to about 100 ug/mg of tissue; from about 1 ng/mg of tissue to about 10 ug/mg of tissue. A mammalian tissue concentration of the substance at a subsequent phase will typically be within a range from about 0.001 ng/mg of tissue to about 600 ug/mg of tissue, preferably from about 1 ng/mg of tissue to about 10 ug/mg of tissue.

The rate of delivery during the initial phase will typically range from about 0.001 ng to about 50 ug per day, usually from about 0.1 ug to about 30 ug per day, more preferably, from about 1 ug per day to about 20 ug per day. The rate of delivery at the subsequent phase may range from about 0.01 ug per day to about 200 ug per day, usually from about lug per day to about 100 ug per day. In one embodiment, the therapeutic capable agent is made available to the susceptible tissue site in a programmed and/or controlled manner with increased efficiency and/or efficacy. Moreover, the present invention provides limited or reduced hindrance to endothelialization of the vessel wall.

The duration of the initial, subsequent, and any other additional phases may vary. For example, the release of the therapeutic capable agent may be delayed from the initial implantation of the device. Typically the delay is sufficiently long to allow the generation of sufficient cellularization or endothelialization at the treated site. Typically, the duration of the initial phase will be sufficiently long to allow initial cellularization or endothelialization at, at least part of the device. Typically, the duration of the initial phase whether being a delayed phase or a release phase, is usually less than about 12 weeks, more usually from about 1 hour to about 8 weeks, more preferably from about 12.hours to about 4 weeks, from about 12 hours to about 2 weeks, from about 1 day to about 2 weeks, or from about 1 day to about 1 week.

The durations of the one or more subsequent phases may also vary, typically being from about 4 hours to about 24 weeks, from about 1 day to about 12 weeks, from about 2 days to about 8 weeks, more preferably in from about of 3 days to about 50 days. In an embodiment, the duration specified relates to a vascular environment. The more than one phase may include similar or different durations, amounts, and/or rates of release. For example, in one scenario, there may be an initial phase of delay, followed by a subsequent phase of release a first subsequent rate, and second subsequent phase at a second subsequent rate of release, and the like.

When the device includes the source including a plurality of compounds (e.g., first therapeutic capable agent and an another compound such as another therapeutic capable agent or enabling compound), the plurality of compounds may be released at different times and/or rates, from the same or different layers when present. Each of the plurality of compounds may be made available independently of another, simultaneous with, or subsequent to the interventional procedure, and may be simultaneous or sequential with one another. For example, a first therapeutic capable agent (e.g., TriptolideTM may be released within a time period of 1 day to 45 days with the second therapeutic capable agent (e.g, mycophenolic acid) released within a time period of 2 days to 3 months, from the time of interventional procedure.

The devices of the present invention may be provided together with instructions for use (IFU), separately or as part of a kit. The kit may include a pouch or any other suitable package, such as a tray, box, tube, or the like, may be used to contain the device and the IFU, where the IFU may be printed on a separate sheet or other media of communication and/or on the packaging itself. In an embodiment of a kit, the kit may also include a mounting hook such as a crimping device and/or an expansible inflation member which may be permanently or releaseably coupled to the device of the present invention. In an embodiment, the kit may comprise the device and an IFU regarding the use of a second compound prior to, concurrent with, or subsequent to, the interventional procedure, and optionally the second compound. In an embodiment, the kit comprises the device and the second compound with or without the IFU for the second compound and/or the device.

In one embodiment, the second compound, may be a therapeutic capable agent, an another compound (e.g., the another therapeutic capable agent and/or the another enabling and/or enhancing compound), or a bio-active compound such as an anti-nausea drug; and being similar or different than that made available to the susceptible tissue site by the device; may be administered prior to, concurrent with, or subsequent to the implanting of the device (e.g., prosthesis) of the present invention.

The second compound may be administered from a pathway similar to or different than that used for the delivery of the therapeutic capable agent as part of the device. By way of example, the second compound may be in the form of a tablet to be taken orally, a transdermal patch to be placed on the patient's skin, subcutaneously, systemically by direct introduction to the blood stream, by way of inhalation, or through any other pathways and bodily orifices. Alternatively, the second compound may be made available to the intracorporeal body by a catheter. In an embodiment, the balloon of a balloon catheter (e.g., perfusion), may be used to perfuse the second compound (e.g., perfusion catheter) into the corporeal body or may be coated with the second compound. The second compound may be made available to the patient continuously or in discrete intervals, prior to, concurrent with, or subsequent to the interventional procedure.

The duration of the availability of the second compound usually may be shorter as compared to that of the therapeutic capable agent. In an embodiment, the another compound may be administered to the patient in a time period ranging from about 200 days prior to about 200 days after the interventional procedure, from about 30 days prior to about 30 days after the interventional procedure, from about 1 day prior to about 30 days after the interventional procedure, from about 200 days prior to about up to the interventional procedure, from about 3 months prior to about up to the interventional procedure, or from about 7 days to about 24 hours prior to the interventional procedure. The duration of the availability of the second compound as measured in the patient's blood may range from about 1 hour to about 120 days, from about 12 hours to about 60 days, or from about 24 hours to about 30 days. Examples of bioactive compounds include: antiemetics such as ondansetron (e.g., Zofran™), antinauseant such as dronabinol (e.g., Marinol^{™}) and ganisetron.Hcl (Kytril™).

In one embodiment, the second compound may be the same as the therapeutic capable agent of the device to provide a desired bullous level (e.g., an initial level) of the therapeutic capable agent in the corporeal body. The total amount made available to the susceptible tissue site from the device and the second compound will typically be in a range from about 0.1 ug to about 10 milligrams (mg), preferably in a range from about 10 ug to about 2 mg, more preferably in a range from about 50 ug to about 1.0 mg. In an embodiment the amount of the second compound administered to the patient on a single dose or daily basis, ranges from about 0.5 mg to about 5 g, from about 1 mg to about 3 g, from about 1 g to about 1.5 g, from about 2 g to about 3 g. Examples second compounds being provided at the latter series of doses include, mycophenolic acid, rapamycin; and their respective pro-drugs, metabolites, derivatives, and combinations thereof. In an example mycophenolic acid or rapamycin may be provided as a second compound at individual doses ranging from about 1 g to about 1.5 g, and from about 1 mg to about 3 mg, respectively; and at a daily dose ranging from about 2 g to about 3 g, and from about 2 mg to about 6 mg, respectively.

The expandable structure may incorporate the therapeutic capable agent and/or the optional another compound, by coating, spraying, dipping, deposition, or painting the therapeutic capable agent onto the prosthesis. Usually, the therapeutic capable agent is dissolved in a solvent. Suitable solvents include aqueous solvents (e.g., water with pH buffers, pH adjusters, organic salts, and inorganic salts), alcohols (e.g., methanol, ethanol, propanol, isopropanol, hexanol, and glycols), nitriles (e.g., acetonitrile, benzonitrile, and butyronitrile), amides (e.g., formamide and N-dimethylformamide), ketones, esters, ethers, DMSO, gases (e.g., CO₂), and the like. For example, the prosthesis may be sprayed with or dipped in the solution and dried so that therapeutic capable crystals are left on a surface of the prosthesis. Alternatively, matrix solution including a rate-controlling element material and the therapeutic capable agent may be prepared by dissolving the rate-controlling element material and the therapeutic capable agent. The expandable structure 16 may then be coated with the matrix solution by spraying, dipping, deposition, or painting the matrix onto the prosthesis. By way of example, when the matrix is formed from polymeric material, the matrix solution is finely sprayed on the prosthesis while the prosthesis is rotating on a mandrel. The thickness of the matrix coating may be controlled by the time period of spraying and a speed of rotation of the mandrel. The thickness of the matrix-agent coating is typically in a range from about 0.01 um to about 100 um, preferably in a range from about 0.1 um to about 50 um. Once the prosthesis has been coated with the matrix coating, the stent may be placed in a vacuum or oven to complete evaporation of the solvent.

By way of example, a stainless steel Duraflex^{™} stent (available from Avantec Vascular Corporation, having a place of operation in California), having dimensions of 3.0 mm x 14 mm is sprayed with a solution of 25 mg/ml therapeutic capable agent in a 100% ethanol or methanol solvent. The stent is dried and the ethanol is evaporated leaving the therapeutic capable agent on the stent surface. A 75:25 PLLA/PCL copolymer (sold commercially by POLYSCIENCES) is prepared in 1,4 Dioxane (sold commercially by ALDRICH CHEMICALS). The therapeutic capable agent loaded stent is loaded on a mandrel rotating at 200 rpm and a spray gun (sold commercially by BINKS MANUFACTURING) dispenses the copolymer solution in a fine spray on to the therapeutic capable agent loaded stent as it rotates for a 10-30 second period. The stent is then placed in an oven at 25-35°C up to 24 hours to complete evaporation of the solvent.

In operation, methods of delivering therapeutic capable agents to a susceptible tissue site, comprise providing a luminal prosthesis incorporating features of the present invention as described above. The prosthesis is delivered to a corporeal site, such as a body lumen, including the susceptible tissue site. The prosthesis is implanted within the body lumen. The therapeutic capable agent is made available to the susceptible tissue site over a period of time.

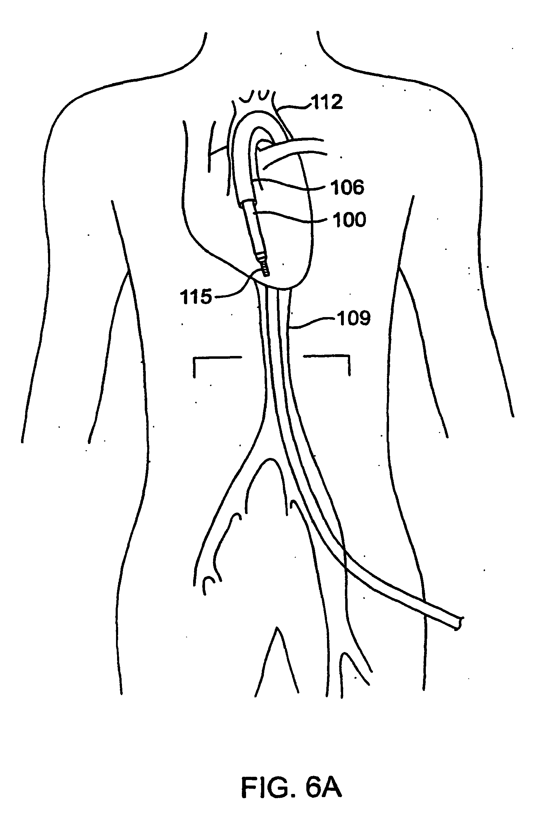

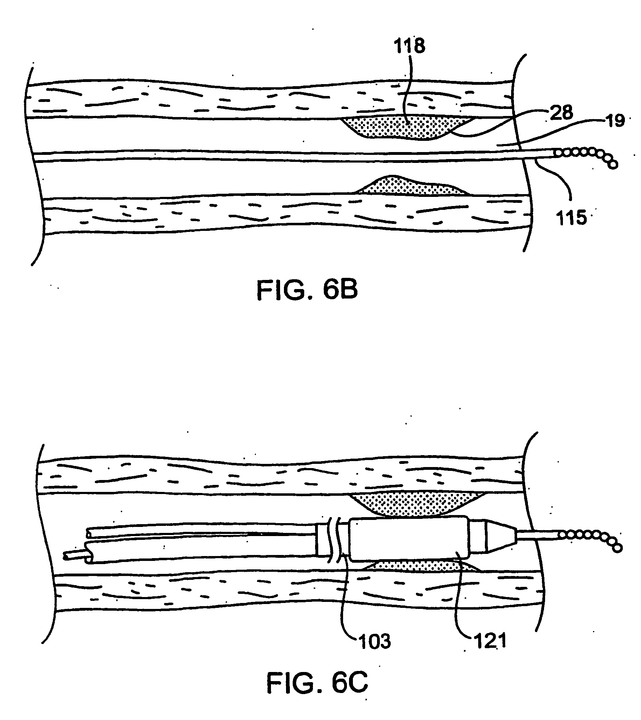

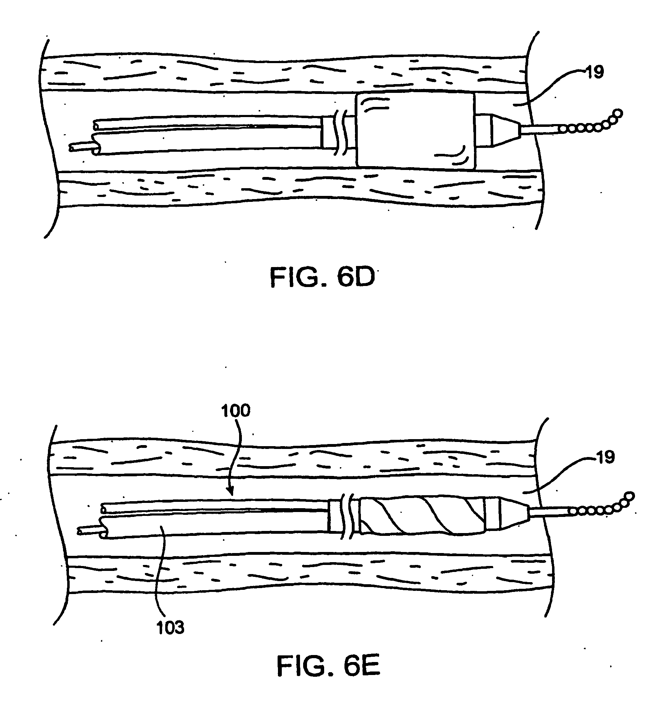

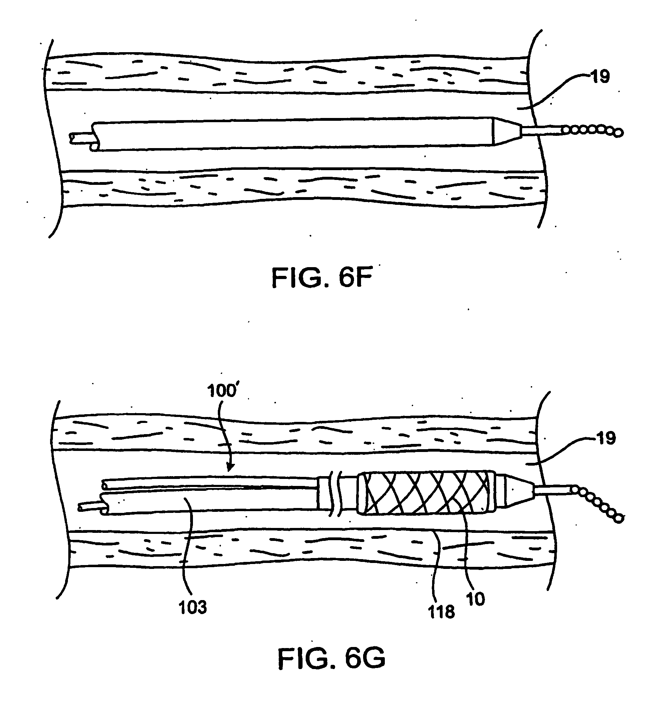

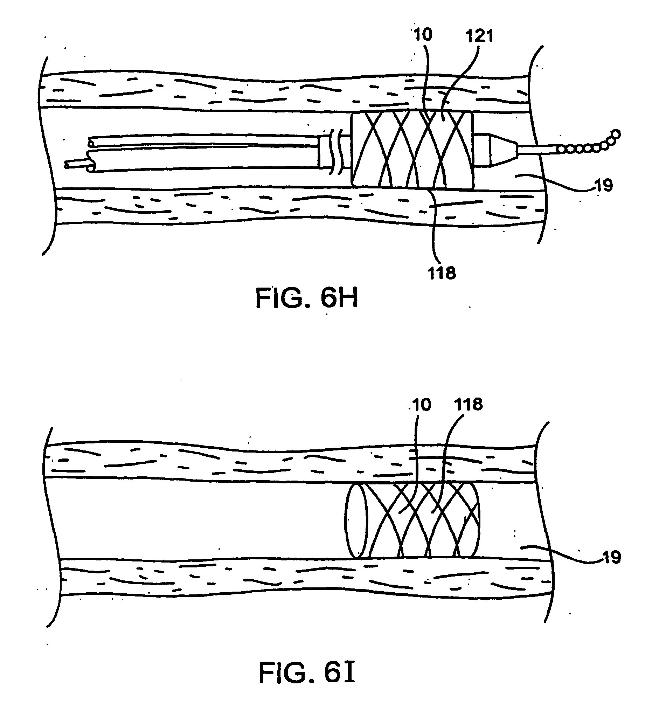

FIGS. 6A-6F, illustrate features of a method for making a therapeutic capable agent available to a susceptible tissue site. As shown in the figures, an intravasculature balloon catheter 100 having a tubular body 103 is introduced through a guiding catheter 106 via hemostatic valve and sheath (not shown) and through the femoral artery 106 to the coronary vasculature over the aortic arch 112.

A guidewire 115 will usually be positioned at the target site 118 including the susceptible tissue site 22, typically a region of stenosis to be treated by balloon angioplasty. Usually, the balloon catheter 100 and guidewire 115 will be introduced together with the guidewire 115 being periodically extended forward of the distal end of the catheter until the target site is reached.

Once at the target site 118, a balloon 121 is inflated, in order to expand the occlusion at the target site 118. After the balloon angioplasty treatment is completed, the balloon 121 will be deflated, with guidewire 115 remaining in place. The balloon 121 may then be removed over guidewire 115, again with the guidewire 115 remaining in place. A second balloon assembly 100' including a device 10 according to present invention, is then introduced over the catheter body. After the second balloon assembly 100' is in place, the device, such as stent 10 which is in place over the balloon assembly may be deployed by inflating balloon 121. After the stent 10 has been properly deployed, the balloon may be deflated and the catheter removed leaving the stent in place.

Methods of treatment, generally, include positioning the source including the at least one therapeutic capable agent and/or optional another compound within the intracorporeal body, concurrently with, or subsequent to, an interventional treatment. More specifically, the therapeutic capable agent may be delivered to a targeted corporeal site (e.g., targeted intracorporeal site) which may include the susceptible tissue site or may provide therapeutic capable agent to the susceptible tissue site, concurrently with or subsequent to the interventional treatment. By way of example, following the dilation of the stenotic region with a dilatation balloon, a device (such as a stent) according to the present invention, is delivered and implanted in the vessel. The therapeutic capable agent may be made available to the susceptible tissue site at amounts which may be sustainable, intermittent, or continuous; at one or more phases and/or rates of delivery.

In an embodiment, the release of the therapeutic capable agent to the susceptible tissue site may be delayed. During the delay period none to small amounts of therapeutic capable agent may be released before the release of substantial amount of therapeutic capable agent. Typically the delay is sufficiently long to allow the sufficient generation of intimal tissue or cellularization, at the treated site to reduce occurrence of thrombotic event.

In one embodiment, delay is sufficiently long to allow the generated neointima to cover at least partially the implanted expandable structure. In an embodiment, the therapeutic capable agent may be released in a time period, as measured from the time of implanting of the device, ranging from about 1 day to about 200 days; from about 1 day to about 45 days; or from about 7 days to about 21 days. In an embodiment, the method further includes directing energy at the device to effect release of the therapeutic capable agent from the device. The energy may include one or more of ultrasound, magnetic resonance imaging, magnetic field, radio frequency, temperature change, electromagnetic, x-ray, heat, vibration, gamma radiation, or microwave. In an embodiment, the therapeutic capable agent may be released at a total amount ranging from about 0.1 ug to about 10 g, from about 0.1 ug to about 10 mg, from about 1 ug to about 10 mg, from about 1 ug to about 2 mg, from about 10 ug to about 2 mg, or from about 50 ug to about 1 mg.

In another embodiment of a method of treatment, the releasing includes release of at least one another compound, as described. The anther compound may be another therapeutic capable agent or an enabling compound, as described. The another compound may be released prior to, concurrent with, subsequent to the therapeutic, capable agent, or sequentially with the therapeutic capable agent.

In an embodiment, a second compound, as described, may be administered to the patient, prior to, concurrent with, or subsequent to the interventional procedure. The second compound may be administered from pathways, at time periods, and at levels, as described.

It should be appreciated that depending on the nature of the site under treatment, the device of the present invention may be introduced to the site during the introduction of the first balloon catheter without the need for pre-dilatation.

In general, it will be possible to combine elements of the differing prostheses and treatment methods as described above. For example, a prosthesis having reservoir means for releasing therapeutic capable agents may further incorporate a rate-controlling barrier. Additionally, methods of the present invention may combine balloon angioplasty and/or other interventional treatments to resolve a stenotic site with the presently described luminal therapeutic capable delivery treatments.

### EXAMPLES

EXAMPLE 1 - A stainless steel DuraflexTM stent, having dimensions of approximately 3.0 mm x 14 mm was sprayed with a solution of 25 mg/ml therapeutic capable agent in a 100 % ethanol or methanol solvent. The stent was dried and the ethanol was evaporated leaving the therapeutic capable agent on the stent surface. A 75:25 PLLA/PCL copolymer (sold commercially by Polysciences) was prepared in 1,4 Dioxane (sold commercially by Aldrich Chemicals). The therapeutic capable agent coated stent was loaded on a mandrel rotating at 200 rpm and a spray gun (sold commercially by Binks Manufacturing) used to dispense the copolymer solution in a fine spray onto the coated stent, as the stent rotated for approximately a 10-30 second time period. The stent was then placed in an oven at 25-35°C for up to 24 hours to complete the evaporation of the solvent.

EXAMPLE 2 - A Stainless steel Duraflex stent (3.0 x 14 mm) was laser cut from a SS tube. The surface area of the stent for receiving the therapeutic capable agent was increased by increasing the surface roughness of the stent. The surface area and the volume of the stent can be further increased by creating 10 nm wide by 5 nm deep grooves along the links of the stent strut. The grooves were created in those stent areas experiencing low stress during expansion so as not to compromise the stent radial strength. The drug was loaded onto the stent and in the stent grooves by dipping or spraying the stent in the therapeutic capable agent solution prepared in low surface tension solvent such as isopropyl alcohol, ethanol, or methanol. The stent was then dried with the therapeutic capable agent remaining on the stent surface, and in the grooves which served as a reservoir for the therapeutic capable agent. Parylene was then vacuum deposited on the stent to serve as a rate-controlling barrier. The drug was eluted from the stent over a period of time in the range from 1 day to 45 days.

EXAMPLE 3 - A therapeutic capable agent was dissolved in methanol, then sprayed onto the stent. The stent was left to dry with the solvent evaporating from the stent leaving the therapeutic capable agent on the stent. A matrix or barrier (silicone, polyurethane, polytetrafluorethylene, parylast, parylene) was sprayed or deposited on the stent covering the therapeutic capable agent. The amount of therapeutic capable agent varied from about 100 micrograms to 2 milligrams, with release rates from 1 day to 45 days.

EXAMPLE 4 - A matrix solution including the matrix polymer and a therapeutic capable agent was coated onto a stent, as described in Example 2. The stent was then coated or sprayed with a top coat of a rate-controlling barrier (and/or a matrix material without a drug so as to act as a rate-controlling barrier). Alternatively, the therapeutic capable agent may be coated on a stent via a rate-controlling barrier, and then covered with a top coat (another barrier or matrix). Use of top coats provides further control of release rate, improved biocompatibility, and/or resistance to scratching and cracking upon stent delivery or expansion.

EXAMPLE 5 - The therapeutic capable agent may be combined with a second therapeutic capable agent (cytotoxic drugs, cytostatic drugs, or psoriasis drugs). One agent is in or coupled to a first coat while other agent is in or coupled to a second coat. The therapeutic capable agent is released for the first 1-3 weeks after being implanted within a vessel while the second therapeutic capable agent is released or continues to be released for a longer period.

EXAMPLE 6 - A combination of multiple therapeutic capable agents that are individually included in different coats can be used as the matrix. The coats may release the multiple agents simultaneously and/or sequentially. The agents may be selected from a therapeutic capable agent class of inhibitors of de novo nucleotide synthesis or from classes of glucocorticosteroids, immunophilin-binding drugs, deoxyspergualin, FTY720, protein drugs, or peptides. This can also apply to any combination of agents from the above classes that are coupled to a stent with the addition of other cytotoxic drugs.

EXAMPLE 7 - A matrix including the therapeutic capable agent, mycophenolic acid, and matrix polymer, CAB (cellulose acetate butyrate); at a mycophenolic acid loading of 70 % to 80% by weight was prepared by dissolving the therapeutic capable agent in acetone at 15 mg/ml concentration, dissolving CAB in acetone at 15 mg/ml concentration, and thereafter mixing together the mycophenolic acid and CAB solutions in 3:1 portion matrix solution. The amount of therapeutic capable agent varied from about 0.1 microgram to about 2 mg, preferably, at 600 microgram. The matrix solution was then coated onto two sets of stents (Sets A and B) by spraying them with an atomizer sprayer (EFD manufacturer) while each stent was rotated. Each stent was allowed to let dry. One matrix-coated stent was then coated with parylene as the rate-controlling barrier (about 1.1 um) using methods similar to those described in Example 2. Orifices were created on the top surface (parylene rate-controlling barrier) of the stent of Set B by subjecting the surface to laser beams or needle. The orifice size can range from about 0.1 um to about 100 um in diameter. The orifice in Set B stent was about 10 um in diameter. An orifice can be about 0.0762 mm to about 50.8 mm (about 0.003 to about 2 inches) apart from the next orifice (measured as the curvilinear distance as you trace along the stent strut pattern).

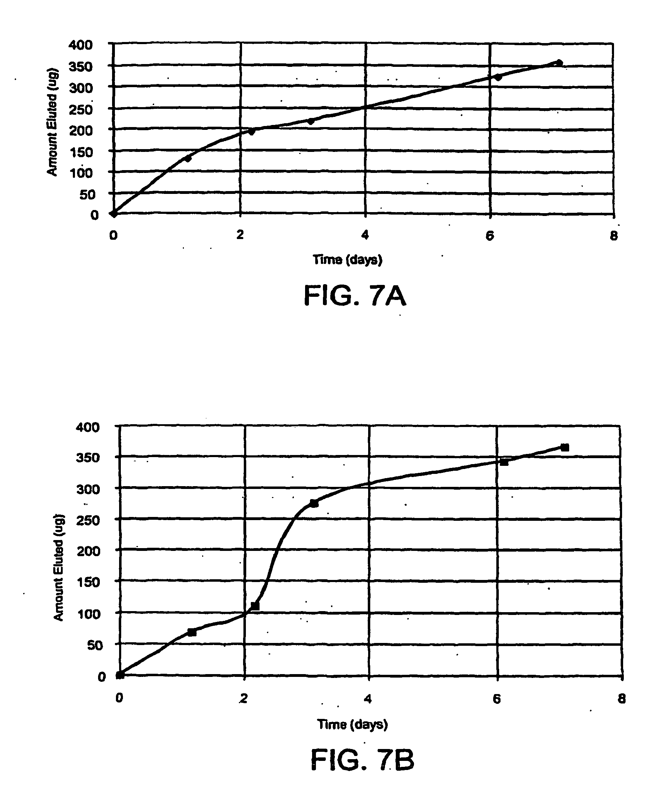

The mycophenolic acid loaded stents were placed in an elution solution of porcine serum and allowed to age for a period of 1 to 7 days. Samples from the serum were taken at regular time intervals and analyzed by HPLC. As can be seen from the data represented in FIGS. 7A and 7B (corresponding to stent sets A and B, respectively), Stent Set A showed a linear release rate for the mycophenolic acid while stent Set B showed a relatively slow linear release rate at the initial phase, followed by a relatively more rapid release in the subsequent phase.

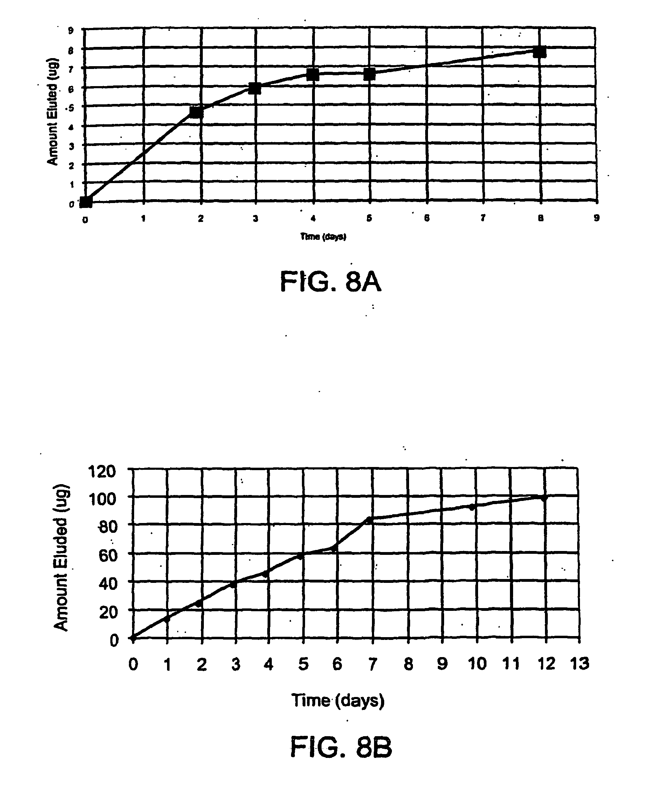

EXAMPLE 8 - Two sets of stents, Sets A and B, were coated with 250 and 300 µg of mycophenolic acid, respectively, according to Example 2. Set A was then coated with 1.7 micron of parylene as the rate-controlling barrier. Set B was first coated with mycophenolic acid followed by a subsequent coating of methylprednisolone as the rate-limiting matrix material, and thereafter coated with 1.3 micron of parylene. The coated stents were then subjected to in vitro elution test as described in Example 7, and the amount of mycophenolic acid eluted was measured. As can be seen from the data represented in FIGS. 8A and 8B (corresponding to stent Sets A and B, respectively), both Sets showed a relatively fast linear release of the mycophenolic acid in the initial phase followed by a relatively slower release in the subsequent phase. This may suggest that the more hydrophobic methylprednisolone may act as a rate-controlling element for the more water soluble mycophenolic acid, and can act to control the release rate of mycophenolic acid along with the Parylene coating. This is useful when the diseased area needs a large bolus of the drug initially and then a sustained slower release.

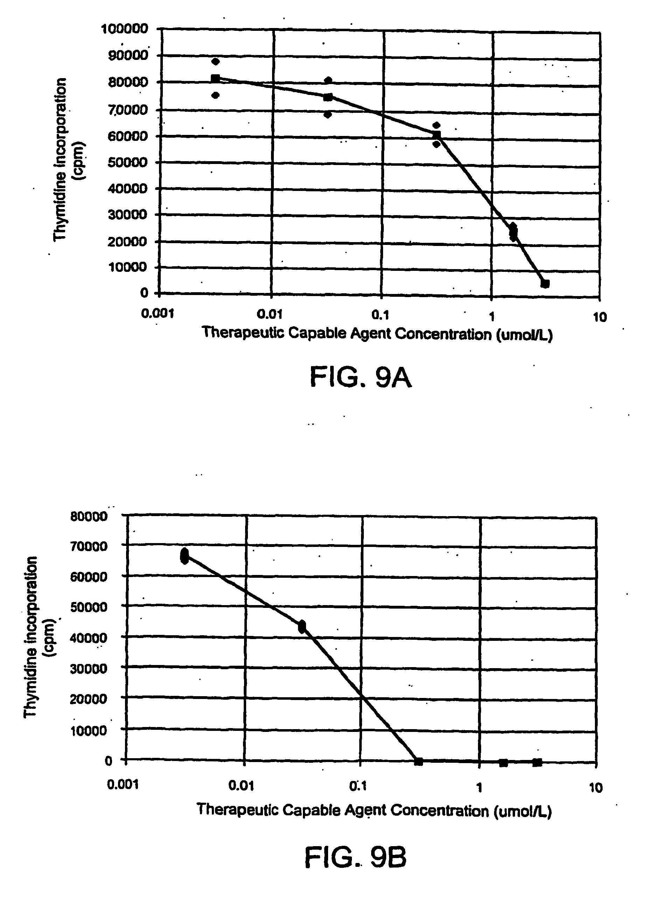

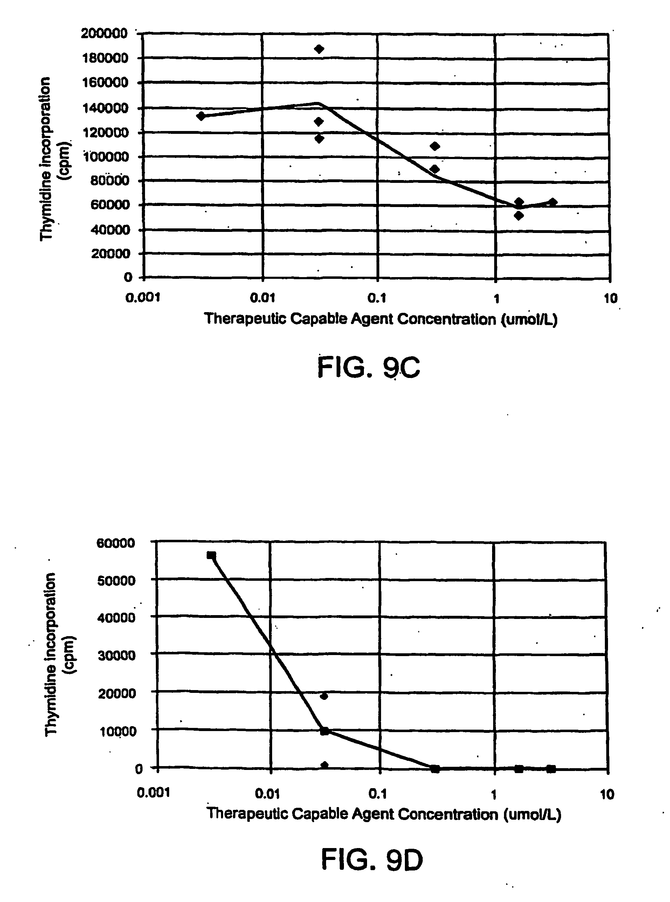

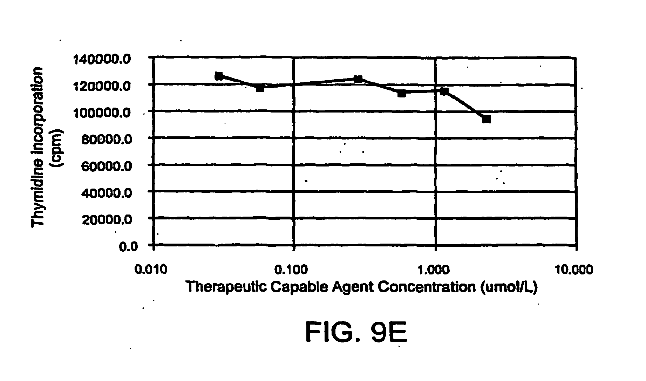

EXAMPLE 9 - In order to assess the effect of therapeutic capable agents of the present invention on cell cultures, samples of 5 sets of therapeutic capable agents, as listed below, in varying concentrations were prepared and added to different groups of porcine smooth muscle cell cultures according to standard procedures. Set A, B, C, D, and E corresponded to therapeutic capable agent sets: Mycophenolic acid & Dexamethasone; Mycophenolic acid & Triptolide; Wortmannin and Methotrexate; Triptolide; Mycophenolate Mofetil; respectively. The amount of incorporated thymidine for the different samples of varying concentrations (0.003, 0.031, 0.31, 1.6, and 3.1 micromolar) was measured. As can be seen from the data represented in FIGS. 9A-9E (corresponding to Sets A-E, respectively) the IC50 (defined as the concentration at which 50% of the cells are prevented from proliferating) for the various sets occurred at different concentrations. As can further be noted, Mycophenolate Mofetil (reference E) may not be as effective in the absence of a bio-condition (e.g., subject to bodily fluids such as blood).

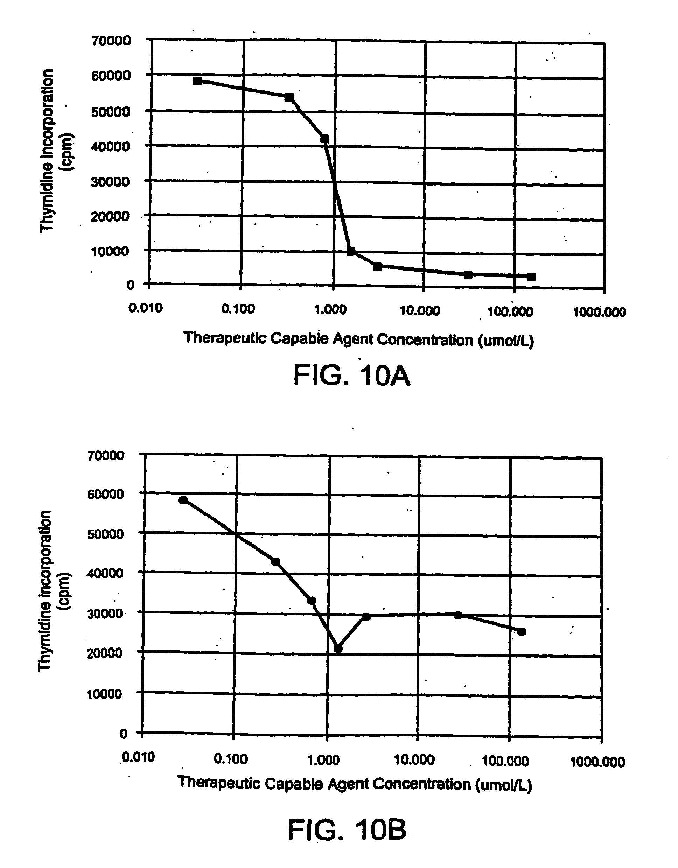

EXAMPLE 10 - In another group of therapeutic capable agents, the amount of incorporated thymidine for samples of varying concentrations (0.003, 0.031, 0.31, 1.6, 3.1, 31, and 156 micromolar) was measured. As can be seen from the data represented in FIGS. 10A-10B, and corresponding to Mycophenolic acid and Methylprednisolone, respectively, the IC50 for these therapeutic capable agent was 1.0 micromolar.

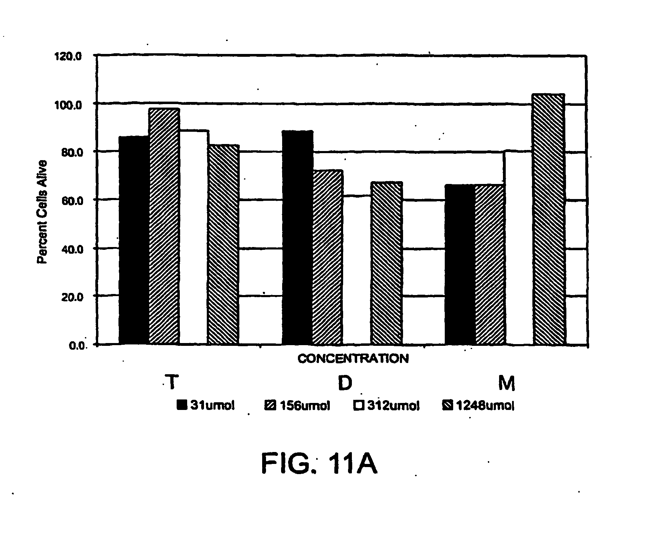

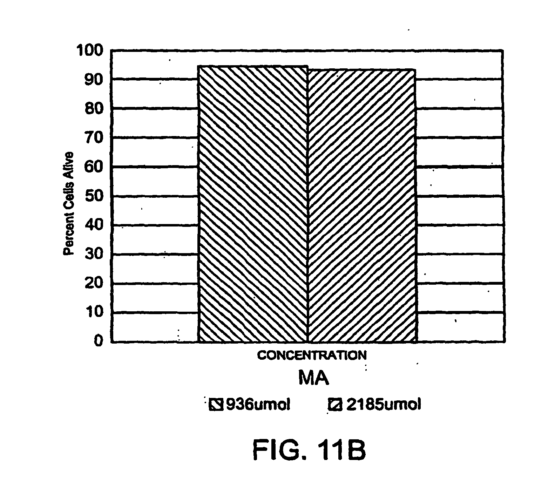

EXMAPLE 11 - In order to assess the effect of various therapeutic capable agents, cell cultures were subjected to some therapeutic capable agents, using methods similar to those described in Examples 9 and 10. As can be seen from data represented in FIGS. 11A-11B, and corresponding, respectively, to Triptolide (T), Dexamethasone (D), Methotrexate (M); and Mycophenolic Acid (MA); the therapeutic capable agents did not lead to significant cell death. In addition, it can be seen that at the IC50 concentrations, most of the cells were alive yet 50% proliferating.

EXAMPLE 12 - A therapeutic capable agent, mycophenolic acid, was prepared by dissolving the therapeutic capable agent in acetone at 15 mg/ml concentration. The amount of therapeutic capable agent varied from about 0.1 ug to about 2 mg, preferably, at 600 ug. The drug solution was then coated onto or over a stent as described in Example 8 by spraying them with an atomizer sprayer (EFD manufacturer) while the stent was rotated. The stent was allowed to let dry. The stent was then placed over the tri-fold balloon on a PTCA catheter and crimped thereon. After crimping, the drug remained intact and attached to the stent. Expansion of the stent against a simulated Tecoflex vessel showed no cracking of the drug. Exposure of fluid flow over the stent before stent deployment against the simulated vessel did not result in drug detachment from the stent.

Although certain preferred embodiments and methods have been disclosed herein, it will be apparent from the foregoing disclosure to those skilled in the art that variations and modifications of such embodiments and methods may be made without departing from the scope of the appended claims. Therefore, the above description should not be taken as limiting the scope of the invention which is defined by the appended claims.

## Claims

1. A device for intracorporeal use, comprising:
an expandable structure (16);
at least one source (25) of at least one therapeutic capable agent associated with the structure (16); and
at least one rate-controlling element layer (43) disposed adjacent at least a portion of the expandable structure (16) or the source (25), the device being configured to release the therapeutic capable agent at multiple rates including an initial phase having a rate of release ranging from about 0 to about 99% of a subsequent rate of release in a subsequent phase.

2. The device of Claim 1, wherein the device is configured to release the at least one therapeutic capable agent to a body lumen or organ within an intracorporeal body to inhibit smooth muscle cell proliferation.

3. The device of Claim 1 or Claim 2, wherein the expandable structure (16) comprises a stent, graft, or a scaffold formed at least in part from an open lattice.

4. The device as in any of claims 1-3, wherein the expandable structure has a luminal (34) and a tissue (31) facing surface, and wherein the therapeutic capable agent is associated with at least one of the lumuinal (34) or tissue (31) facing surfaces.

5. The device as in any of claims 1-4, wherein the therapeutic capable agent comprises at least one agent selected from the group consisting of immunosuppressants, anti-inflammatories, anti-proliferatives, anti-migratory agents, anti-fibrotic agents, proapoptotics, calcium channel blockers, anti-neoplastics, antibodies, antithrombotic agents, anti-platelet agents, IIb/IIIa agents, antiviral agents, and a combination thereof.

6. The device as in any of claims 1 - 5, wherein the therapeutic capable agent is at least one agent selected from the group consisting of mycophenolic acid, rapamycin, mizoribine, methylprednisolone, mycophenolate mofetil, dexamethasone, hydroxyurea, cyclophosphamide, cyclosporine, daclizumab, azathioprine, prednisone, derivatives and combinations thereof.

7. The device as in any of claims 1- 6, wherein the source (25) further includes another compound.

8. The device as in Claim 7, wherein the device is configured to release the another compound prior to, concurrent with, or subsequent to the release of the therapeutic capable agent.

9. The device as in any of claims 1 - 8, wherein the device is configured to release the therapeutic capable agent at a release rate so as to provide a sustainable level of therapeutic capable agent to a susceptible tissue site.

10. The device as in any one of claims 1-9, wherein the device is configured to release the therapeutic capable agent at a rate between about 0.001 µg to about 200 µg/day.

11. The device as in any one of claims 1-10, wherein the device is configured to release the therapeutic capable agent at a total amount ranging from about 0.1 µg to about 10 g.

12. The device as in any of claims 1-11, wherein the initial rate of release is in a range between about 0 to about 50 µg/g/day, and the subsequent rate of release is in a range between about 0.01 µg to about 200 µg/day.

13. The device as in any of claims 1-12, wherein the initial rate is in a range between about 10 to about 300 µg/day, and the subsequent rate of release is in a range between about 0.1 to about 100 µg/day.

14. The device as in any of claims 1-13, wherein a time duration of the initial rate is configured to last less than about 24 weeks.

15. The device as in any of claims 1-13, wherein a time duration of the subsequent rate is configured to last from about 1 hour to about 50 weeks.

16. The device as in any of claims 1-11, wherein the device is configured to deliver the therapeutic capable agent at a rate to a susceptible tissue site of a mammalian intracorporeal body to effectuate a mammalian tissue concentration ranging from about 0.001 ng of therapeutic capable agent / mg of tissue to about 100 µg of therapeutic capable agent / mg of tissue.

17. The device as in any of claims .1-11, wherein the device is configured to release the therapeutic capable agent at a rate to a mammalian intracorporeal body to effectuate a mammalian blood concentration ranging from about 1 ng of therapeutic capable agent / ml of blood to about 50 µg of therapeutic capable agent /ml of blood.

18. The device as in any one of claims 1-11, wherein the source is configured to delay release of the therapeutic capable agent, wherein the delay is sufficiently long to allow formation of a sufficient amount of cellularization, endothelization, or fibrin deposition at a susceptible tissue site or on the device.

19. The device of any of claims 1-18, wherein at a least a portion of the rate-controlling element (43) forms a matrix with the therapeutic capable agent.

20. The device of any of claims 1-18, wherein at least a portion of the rate-controlling element (43) forms a layer adjacent at least a portion of the therapeutic capable agent.

21. The device of any preceding claim, wherein the therapeutic capable agent is released by surface degradation, bulk degradation, diffusion, or hydrolysis of the rate-controlling element (43).

22. The device of any preceding claim, wherein the rate-controlling element is formed from a material selected from the group consisting of parylene, parylast, polyurethane, polyethylenes imine, cellulose acetate butyrate, ethylene vinyl alcohol copolymer, silicone, polytetrafluorethylene (PTFE), poly (methyl methacrylate butyrate), poly-N-butyl methacrylate, poly (methyl methacrylate), poly 2-hydroxy ethyl methacrylate, poly ethylene glycol methacrylates, poly vinyl chloride, poly(dimethyl siloxane), poly(tetrafluoroethylene), poly (ethylene oxide), poly ethylene vinyl acetate, poly carbonate, poly acrylamide gels, N vinyl-2-pyrrolidone, maleic anhydride, Nylon, cellulose acetate butyrate (CAB) and the like, including other synthetic or natural polymeric substances; mixtures, copolymers, and combinations thereof.

23. The device of any preceding claim, wherein the rate-controlling element has a thickness ranging from about 10 nm to about 100 µm.

24. The device as in any preceding claim, wherein the source (25) comprises a matrix (40) including the therapeutic capable agent.

25. The device as in any preceding claim, wherein the source is a polymeric material including the therapeutic capable units associated with a polymeric or metallic backbone.

26. The device as in any preceding claim, wherein the source is a reservoir disposed adjacent the expandable structure (16).

27. The device as in any preceding claim, wherein the device is configured to release the therapeutic capable agent in response to an external source of energy.

28. The device as in any preceding claim, wherein the therapeutic capable agent comprises a polymeric material formed at least in part from therapeutic capable agent.

29. The device as in any preceding claim, wherein the therapeutic capable agent is made available to a susceptible tissue site as the expandable structure degrades within the intracorporal body over time.

30. The device as in any preceding claim, wherein the device further comprises a bio-compatible outer layer.

31. The device as in any preceding claim, wherein the source is associated with the expandable structure by coating, spraying, dipping, vapor deposition, plasma deposition, or painting of the source onto or in the expandable structure.

## Patentansprüche

1. Eine Vorrichtung zum intrakorporalen Gebrauch, umfassend:
eine ausdehnbare Struktur (16);
mindestens eine Quelle (25) mindestens eines therapeutisch geeigneten mit der Struktur (16) verbundenen Mittels; und
mindestens eine ratenkontrollierende Elementschicht (43), die neben zumindest einem Anteil der ausdehnbaren Struktur (16) oder der Quelle (25) angeordnet ist, wobei die Vorrichtung zum Freigeben des therapeutisch geeigneten Mittels bei mehreren Raten, die eine anfängliche Phase einschließen, welche eine Rate der Freigabe von ungefähr 0 bis ungefähr 99 % einer nachfolgenden Rate der Freigabe in einer darauf folgenden Phase aufweist, ausgelegt ist..

2. Vorrichtung nach Anspruch 1, wobei die Vorrichtung dafür ausgelegt ist, das mindestens eine therapeutisch geeignete Mittel an ein Körperlumen oder Organ innerhalb eines intrakorporalen Körpers freizugeben, um die Proliferation glatter Muskelzellen zu hemmen.

3. Vorrichtung nach Anspruch 1 oder Anspruch 2, wobei die ausdehnbare Struktur (16) einen Stent, ein Transplantat oder ein Gerüst, welches zumindest teilweise aus einem offenen Gitter gebildet ist, umfasst.

4. Vorrichtung nach einem der Ansprüche 1 - 3, wobei die ausdehnbare Struktur eine dem Lumen (34) und eine dem Gewebe (31) zugewandte Oberfläche aufweist, und wobei das therapeutisch geeignete Mittel mit mindestens einer dem Lumen (34) oder dem Gewebe (31) zugewandten Oberflächen verbunden ist.

5. Vorrichtung nach einem der Ansprüche 1 - 4, wobei das therapeutisch geeignete Mittel mindestens ein Mittel, welches aus der Gruppe bestehend aus Immunosuppressiva, Entzündungshemmern, Wachstumshemmern, Wanderungshemmern, Anti-Fibrosis-Mitteln, proapoptotischen Mitteln, Kalziumkanalblockern, Antineoplastica, Antikörpern, antithrombotische Mitteln, Antiplatelet Mitteln, IIb/IIIa-Mitteln, Anti-Virenmitteln und Kombinationen daraus ausgewählt ist, umfasst.

6. Vorrichtung nach einem der Ansprüche 1 - 5, wobei das therapeutisch geeignete Mittel mindestens ein Mittel ist, welches aus der Gruppe bestehend aus Mycophenolsäure, Rapamycin, Mizoribin, Methylprednisolon, Mycophenolat Mofetil Dexamethason, Hydroxyharnstoff, Cyclophosphamid, Ciclosporin, Daclizumab, Azathioprin, Prednison, Derivaten und Kombinationen daraus, ausgewählt ist.

7. Vorrichtung nach einem der Ansprüche 1 - 6, wobei die Quelle (25) eine weitere Verbindung beinhaltet.

8. Vorrichtung nach Anspruch 7, wobei die Vorrichtung zum Freigeben der weiteren Verbindung vor, gleichlaufend mit oder nachfolgend zu dem Freigeben des therapeutisch geeigneten Mittels ausgelegt ist.

9. Vorrichtung nach einem der Ansprüche 1 - 8, wobei die Vorrichtung zum Freigeben des therapeutisch geeigneten Mittels mit einer Freigaberate ausgelegt ist, um so ein anhaltendes Niveau des therapeutisch geeigneten Mittels für eine empfängliche Gewebestelle bereitzustellen.

10. Vorrichtung nach einem der Ansprüche 1 - 9, wobei die Vorrichtung zum Freigeben des therapeutisch geeigneten Mittels mit einer Rate zwischen ungefähr 0,001 µg bis ungefähr 200 µg/Tag ausgelegt ist.

11. Vorrichtung nach einem der Ansprüche 1 -10, wobei die Vorrichtung zum Freigeben des therapeutisch geeigneten Mittels in einer Gesamtmenge im Bereich von ungefähr 0,1 µg bis ungefähr 10 g ausgelegt ist.

12. Vorrichtung nach einem der Ansprüche 1-11, wobei die anfängliche Rate der Freigabe in einem Bereich von ungefähr 0 bis ungefähr 50 µg/Tag ist und die nachfolgende Rate der Freigabe in einem Bereich von ungefähr 0,01 µg bis ungefähr 200 µg/Tag ist.

13. Vorrichtung nach einem der Ansprüche 1 -12, wobei die anfängliche Rate in einem Bereich zwischen ungefähr 10 bis ungefähr 300 µg/Tag ist und die nachfolgende Rate der Freigabe in einem Bereich zwischen ungefähr 0,1 bis ungefähr 100 µg/Tag ist.

14. Vorrichtung nach einem der Ansprüche 1-13, wobei eine Zeitdauer der anfänglichen Rate ausgelegt ist, weniger als ungefähr 24 Wochen zu dauern.

15. Vorrichtung nach einem der Ansprüche 1 - 13, wobei eine Zeitdauer der nachfolgenden Rate ausgelegt ist, von ungefähr 1 Stunde bis ungefähr 50 Wochen zu dauern.

16. Vorrichtung nach einem der Ansprüche 1 -11, wobei die Vorrichtung dafür ausgelegt ist, das therapeutisch geeignete Mittel mit einer Rate zu einer empfänglichen Gewebestelle eines intrakorporalen Säugetierkörpers zu liefern, um eine Säugetier-Gewebekonzentration im Bereich von ungefähr 0,001 ng des therapeutisch geeigneten Mittels / mg Gewebe bis ungefähr 100 µg an therapeutisch geeignetem Mittel / mg Gewebe herbeizuführen.

17. Vorrichtung nach einem der Ansprüche 1 - 11, wobei die Vorrichtung dafür ausgelegt ist, das therapeutisch geeignete Mittel mit einer Rate an einen intrakorporalen Säugetierkörper freizugeben, um eine Säugetier-Blutkonzentration im Bereich von ungefähr 1 ng des therapeutisch geeigneten Mittels / ml an Blut bis ungefähr 50 µg des therapeutisch geeigneten Mittels / ml an Blut herbeizuführen.

18. Vorrichtung nach einem der Ansprüche 1 - 11, wobei die Quelle dafür ausgelegt ist, die Freigabe des therapeutisch geeigneten Mittels zu verzögern, wobei die Verzögerung ausreichend lang ist, um die Bildung eines ausreichenden Anteils an Zellularisierung, Endothelisierung oder Fibrindeposition an einer empfänglichen Gewebestelle oder auf der Vorrichtung zu gestatten.

19. Vorrichtung nach einem der Ansprüche 1 - 18, wobei mindestens ein Anteil des ratenkontrollierenden Elementes (43) mit dem therapeutisch geeigneten Mittel eine Matrix bildet.

20. Vorrichtung nach einem der Ansprüche 1 -18, wobei mindestens ein Anteil des ratenkontrollierenden Elementes (43) eine zumindest einem Anteil des therapeutisch geeigneten Mittels benachbarte Schicht bildet.

21. Vorrichtung nach einem der vorhergehenden Ansprüche, wobei das therapeutisch geeignete Mittel durch Oberflächendegradation, Volumendegradation, Diffusion oder Hydrolyse des ratenkontrollierenden Elementes (43) freigegeben wird.

22. Vorrichtung nach einem der vorhergehenden Ansprüche, wobei das ratenkontrollierende Element aus einem Material, welches aus der Gruppe bestehend aus Parylin, Parylast, Polyurethan, Polyethylenimin, Celluloseacetat-Butyrat, Ethylenvinylalkohol-Copolymer, Silikon, Polytetrafluorethylen (PTFE), Poly-methyl-methacrylat-Butyrat, Poly-N-butyl-Methacrylat, Poly-methyl-Methacrylat, Poly-2-hydroxy-ethyl-Methacrylat, Polyethylenglykol-Methacrylat, Polyvinylchlorid, Polydimethyl-Siloxan, Polytetrafluoroethylen, Polyethylenoxid, Polyethylenvinylacetat, Polycarbonat, Polyacrylamid Gele, N-vinyl-2-Pyrrolidon, Maleinsäureanhydrid, Nylon, Celluloseacetat-Butyrat (CAB) und dergleichen, einschließlich anderer synthetischer oder natürlicher polymerer Substanzen; Mischungen, Copolymere und Kombinationen daraus ausgewählt ist, gebildet ist.

23. Vorrichtung nach einem der vorhergehenden Ansprüche, wobei das ratenkontrollierende Element eine Dicke im Bereich von ungefähr 10 nm bis ungefähr 100 µm aufweist.

24. Die Vorrichtung nach einem der vorhergehenden Ansprüche, wobei die Quelle (25) eine Matrix (40), die das therapeutisch geeignete Mittel beinhaltet, umfasst.

25. Vorrichtung nach einem der vorhergehenden Ansprüche, wobei die Quelle (25) ein polymeres Material, das die therapeutisch geeigneten Einheiten, die mit einem polymeren oder metallischen Rückgrat verbunden sind, beinhaltet.

26. Vorrichtung nach einem der vorhergehenden Ansprüche, wobei die Quelle (25) ein benachbart der ausdehnbaren Struktur (16) angeordnetes Reservoir ist.

27. Vorrichtung nach einem der vorhergehenden Ansprüche, wobei die Vorrichtung ausgelegt ist, das therapeutisch geeignete Mittel in Reaktion auf eine externe Energiequelle freizugeben.

28. Die Vorrichtung nach einem der vorhergehenden Ansprüche, wobei das therapeutisch geeignete Mittel ein zumindest teilweise aus therapeutisch geeigneten Mitteln gebildetes polymeres Material umfasst.

29. Die Vorrichtung nach einem der vorhergehenden Ansprüche, wobei das therapeutisch geeignete Mittel für eine empfängliche Gewebestelle verfügbar wird, während die ausdehnbare Struktur innerhalb des intrakorporalen Körpers mit der Zeit zerfällt.

30. Die Vorrichtung nach einem der vorhergehenden Ansprüche, wobei die Vorrichtung des Weiteren eine biokompatible äußere Schicht umfasst.

31. Die Vorrichtung nach einem der vorhergehenden Ansprüche, wobei die Quelle mit der ausdehnbaren Struktur durch Beschichten, Besprühen, Eintauchen, Aufdampfung, Plasmaabscheidung oder Anstrich der Quelle auf oder in die ausdehnbare Struktur verbunden ist.

## Revendications

1. Dispositif destiné à une utilisation intracorporelle, comprenant :
- une structure extensible (16);
- au moins une source (25) d'au moins un agent à pouvoir thérapeutique associée à la structure (16) ; et
- au moins une couche d'élément de contrôle du taux (43) disposée de façon adjacente au moins d'une portion de la structure extensible (16) ou de la source (25), le dispositif étant configuré pour libérer l'agent à pouvoir thérapeutique à plusieurs taux incluant une phase initiale ayant un taux de libération allant d'environ 0 à environ 99 % d'un taux de libération subséquent dans une phase subséquente.

2. Dispositif selon la revendication 1, dans lequel le dispositif est configuré pour libérer ledit au moins un agent à pouvoir thérapeutique dans une lumière ou un organe de l'organisme au sein d'un corps intracorporel pour inhiber la prolifération des cellules du muscle lisse.

3. Dispositif selon la revendication 1 ou la revendication 2, dans lequel la structure extensible (16) comprend une endoprothèse vasculaire, une greffe, ou une charpente formée au moins en partie dans un réseau ouvert.

4. Dispositif selon l'une quelconque des revendications 1 à 3, dans lequel la structure extensible présente une surface côté lumière (34) et une côté tissu (31), et dans lequel l'agent à pouvoir thérapeutique est associé à au moins l'une des surfaces côté lumière (34) ou côté tissu (31).

5. Dispositif selon l'une quelconque des revendications 1 à 4, dans lequel l'agent à pouvoir thérapeutique comprend au moins un agent choisi dans le groupe consistant en les immunosuppresseurs, les anti-inflammatoires, les anti-proliférants, les agents anti-migrateurs, les agents anti-fribrogènes, les pro-apoptotiques, les inhibiteurs des canaux calciques, les anti-cancéreux, les anticorps, les agents antithrombotiques, les agents antiplaquettaires, les agents IIb/IIIa, les agents anti-viraux, et une combinaison de ceux-ci.

6. Dispositif selon l'une quelconque des revendications 1 à 5, dans lequel l'agent à pouvoir thérapeutique est au moins un agent choisi dans le groupe consistant en l'acide mycophénolique, la rapamycine, la mizoribine, la méthylprednisolone, le mofétilmycophénolate, la dexaméthasone, l'hydroxycarbamide, le cyclophosphamide, la cyclosporine, le daclizumab, l'azathioprine, la prednisone, les dérivés et les combinaisons de ceux-ci.

7. Dispositif selon l'une quelconque des revendications 1 à 6, dans lequel la source (25) inclut en outre un autre composé.

8. Dispositif selon la revendication 7, dans lequel le dispositif est configuré pour libérer ledit autre composé préalablement à, simultanément à, ou subséquemment à la libération de l'agent à pouvoir thérapeutique.

9. Dispositif selon l'une quelconque des revendications 1 à 8, dans lequel le dispositif est configuré pour libérer l'agent à pouvoir thérapeutique à un taux de libération de manière à fournir un niveau durable d'agent à pouvoir thérapeutique à un site de tissu sensible.

10. Dispositif selon l'une quelconque des revendications 1 à 9, dans lequel le dispositif est configuré pour libérer l'agent à pouvoir thérapeutique à un taux d'environ 0,001 µg à environ 200 µg/jour.

11. Dispositif selon l'une quelconque des revendications 1 à 10, dans lequel le dispositif est configuré pour libérer l'agent à pouvoir thérapeutique à raison d'une quantité totale allant d'environ 0,1 µg à environ 10 g.

12. Dispositif selon l'une quelconque des revendications 1 à 11, dans lequel le taux de libération initial est d'environ 0 à environ 50 µg/g/jour, et le taux de libération subséquent est d'environ 0,01 µg à environ 200 µg/jour.

13. Dispositif selon l'une quelconque des revendications 1 à 12, dans lequel le taux initial est d'environ 10 à environ 300 µg/jour, et le taux de libération subséquent est d'environ 0,1 à environ 100 µg/jour.

14. Dispositif selon l'une quelconque des revendications 1 à 13, dans lequel une durée de temps du taux initial est configurée pour durer moins d'environ 24 semaines.

15. Dispositif selon l'une quelconque des revendications 1 à 13, dans lequel une durée de temps du taux subséquent est configurée pour durer d'environ 1 heure jusqu'à environ 50 semaines.

16. Dispositif selon l'une quelconque des revendications 1 à 11, dans lequel le dispositif est configuré pour délivrer l'agent à pouvoir thérapeutique à un taux à un site de tissu sensible d'un corps intracorporel de mammifère pour générer une concentration dans le tissu de mammifère allant d'environ 0,001 ng d'agent à pouvoir thérapeutique/mg de tissu jusqu'à environ 100 µg d'agent à pouvoir thérapeutique/mg de tissu.

17. Dispositif selon l'une quelconque des revendications 1 à 11, dans lequel le dispositif est configuré pour libérer l'agent à pouvoir thérapeutique à un taux dans le corps intracorporel de mammifère pour générer une concentration dans le sang de mammifère allant d'environ 1 ng d'agent à pouvoir thérapeutique/ml de sang jusqu'à environ 50 µg d'agent à pouvoir thérapeutique/ml de sang.

18. Dispositif selon l'une quelconque des revendications 1 à 11, dans lequel la source est configurée pour retarder la libération de l'agent à pouvoir thérapeutique, dans lequel le retard est suffisamment long pour permettre la formation d'une quantité suffisante de structure cellulaire, de structure endothéliale, ou de dépôt de fibrine à un site de tissu sensible ou sur le dispositif.

19. Dispositif selon l'une quelconque des revendications 1 à 18, dans lequel au moins une portion de l'élément de contrôle du taux (43) forme une matrice avec l'agent à pouvoir thérapeutique.

20. Dispositif selon l'une quelconque des revendications 1 à 18, dans lequel au moins une portion de l'élément de contrôle du taux (43) forme une couche adjacente au moins d'une portion de l'agent à pouvoir thérapeutique.

21. Dispositif selon l'une quelconque des revendications précédentes, dans lequel l'agent à pouvoir thérapeutique est libéré par dégradation de surface, dégradation globale, diffusion, ou hydrolyse de l'élément de contrôle du taux (43).

22. Dispositif selon l'une quelconque des revendications précédentes, dans lequel l'élément de contrôle du taux est formé dans un matériau choisi dans le groupe consistant en parylène, parylast, polyuréthanne, polyéthylène-imines, acétobutyrate de cellulose, copolymère éthylène-alcool vinylique, silicone, polytétrafluoréthylène (PTFE), poly(méthacrylate de méthyle - butyrate), méthacrylate de poly-N-butyle, poly(méthacrylate de méthyle), polyméthacrylate de 2-hydroxy-éthyle, méthacrylates de polyéthylèneglycol, polychlorure de vinyle, poly(diméthylsiloxane), poly(tétrafluoréthylène), poly(éthylène oxyde), polyéthylène-acétate de vinyle, polycarbonate, gels de polyacrylamide, N-vinyl-2-pyrrolidone, anhydride maléique, nylon, acétobutyrate de cellulose (CAB) et d'autres, incluant d'autres substances polymériques synthétiques ou naturelles ; les mélanges, les copolymères, et les combinaisons de ceux-ci.

23. Dispositif selon l'une quelconque des revendications précédentes, dans lequel l'élément de contrôle du taux a une épaisseur allant d'environ 10 nm jusqu'à environ 100 µm.

24. Dispositif selon l'une quelconque des revendications précédentes, dans lequel la source (25) comprend une matrice (40) incluant l'agent à pouvoir thérapeutique.

25. Dispositif selon l'une quelconque des revendications précédentes, dans lequel la source est un matériau polymérique incluant les unités à pouvoir thérapeutique associées à un squelette polymérique ou métallique.

26. Dispositif selon l'une quelconque des revendications précédentes, dans lequel la source est un réservoir disposé de façon adjacente de la structure extensible (16).

27. Dispositif selon l'une quelconque des revendications précédentes, dans lequel le dispositif est configuré pour libérer l'agent à pouvoir thérapeutique en réponse à une source d'énergie externe.

28. Dispositif selon l'une quelconque des revendications précédentes, dans lequel l'agent à pouvoir thérapeutique comprend un matériau polymérique formé au moins en partie par l'agent à pouvoir thérapeutique.

29. Dispositif selon l'une quelconque des revendications précédentes, dans lequel l'agent à pouvoir thérapeutique est rendu disponible sur un site de tissu sensible à mesure que la structure extensible se dégrade au sein du corps intracorporel avec le temps.

30. Dispositif selon l'une quelconque des revendications précédentes, dans lequel le dispositif comprend en outre une couche externe biocompatible.

31. Dispositif selon l'une quelconque des revendications précédentes, dans lequel la source est associée à la structure extensible par revêtement, pulvérisation, immersion, dépôt par évaporation sous vide, revêtement par projection plasma, ou peinture de la source sur ou dans la structure extensible.
